(19) Europäisches Patentamt
European Patent Office
Office européen des brevets

(11) EP 4 063 318 A1

(12) # EUROPEAN PATENT APPLICATION
published in accordance with Art. 153(4) EPC

(43) Date of publication:
**28.09.2022 Bulletin 2022/39**

(21) Application number: **20887237.4**

(22) Date of filing: **16.10.2020**

(51) International Patent Classification (IPC):
***B82Y 30/00*** *(2011.01)* ***G01N 27/12*** *(2006.01)*
***B01J 23/648*** *(2006.01)*

(52) Cooperative Patent Classification (CPC):
**B01J 23/648; B82Y 30/00; G01N 27/12**

(86) International application number:
**PCT/JP2020/039138**

(87) International publication number:
**WO 2021/095440 (20.05.2021 Gazette 2021/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.11.2019 JP 2019206038**

(71) Applicants:
- **National Institute for Materials Science**
**Tsukuba-shi, Ibaraki 305-0047 (JP)**
- **National Institute Of Advanced Industrial Science and Technology**
**Chiyoda-ku**
**Tokyo 100-8921 (JP)**

(72) Inventors:
- **ISHIHARA, Shinsuke**
**Tsukuba-shi, Ibaraki 305-0047 (JP)**
- **NAKANISHI, Takashi**
**Tsukuba-shi, Ibaraki 305-0047 (JP)**
- **HONG, Dachao**
**Tsukuba-shi, Ibaraki 305-8560 (JP)**

(74) Representative: **Markfort, Iris-Anne Lucie**
**Lorenz & Kollegen**
**Patentanwälte Partnerschaftsgesellschaft mbB**
**Alte Ulmer Straße 2**
**89522 Heidenheim (DE)**

(54) **ALKENE DETECTION GAS SENSOR, AND SYSTEM USING SAME**

(57) [Object] To provide a compact and reusable alkene-detection gas sensor that detects an alkene and a system using the same.

[Solving Means] An alkene-detection gas sensor that detects an alkene in a sample gas according to the present invention includes: a first reaction unit that contains a palladium catalyst and oxidizes an alkene in a sample gas to convert the alkene into an aldehyde and/or a ketone; a second reaction unit that contains hydroxylamine salts and reacts with the aldehyde and/or ketone converted in the first reaction unit to generate an acid; and a response unit that includes an electrode supporting a semiconductor material of which an electrical resistance value changes by the generated acid, in which the palladium catalyst, the hydroxylamine salts, and the semiconductor material are separated from each other.

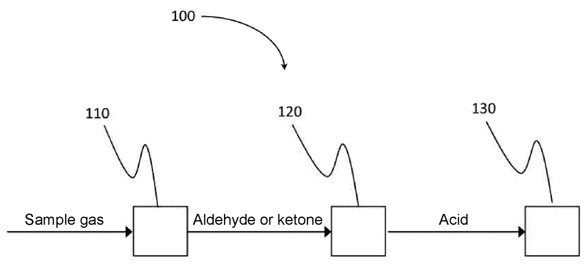


FIG.1

EP 4 063 318 A1

## Description

### Technical Field

The present invention relates to an alkene-detection gas sensor that detects an alkene gas and a system using the same.

### Background Art

The alkene is a general term for chemical substances having a carbon-carbon double bond. the alkene is a raw material for synthesizing various chemical substances (e.g., polyethylene, ethanol), and is used on a large scale industrially.

Further, ethylene that is the simplest alkene functions as a plant hormone, and is used for force-ripening because it promotes the ripening of fruits and vegetables. Meanwhile, since excess ethylene released from fruits and vegetables promotes putrefaction, it is desirable to appropriately control the concentration of ethylene contained in the atmosphere in the preservation and distribution of fruits and vegetables. Examples of the method of measuring the concentration of ethylene include a detector tube and gas chromatography, but they have problems in repeated measurement and miniaturization. Further, although a compact sensor that electrochemically detects an ethylene gas (e.g., Sensor XS EC Organic Vapors 68 09 115 manufactured by Drager) is commercially available, many of them respond to organic chemical substance vapor other than ethylene and have a problem in selectively detecting an alkene (particularly, ethylene).

For example, the concentration of ethylene used for force-ripening is 4 to 20 ppm for potatoes, 300 to 1000 ppm for bananas, approximately 10 ppm for kiwi fruits, and a compact ethylene sensor capable of highly-selectively and repeatedly detecting these concentrations has a high possibility for application.

Further, in recent years, a sensor that uses carbon nanotube to detect an ethylene gas has been developed (see, for example, Patent Literature 1). In accordance with the sensor in Patent Literature 1, a mixture of carbon nanotube and a transition metal complex is used, the phenomenon that ethylene molecules coordinate to the transition metal complex changes the chemical environment in the vicinity of the surface of the carbon nanotube, and the conductivity of the carbon nanotube is changed. The amount of change in conductivity can be easily detected by a compact device (e.g., electric resistance meter) as the amount of change in the current value flowing under a constant voltage. However, the transition metal complex adsorbs also molecules (e.g., tetrahydrofuran, acetonitrile, acetaldehyde, water) other than ethylene having coordinating properties to change the conductivity of the carbon nanotube, and thus has a problem in selective detection of ethylene.

Meanwhile, a compact sensor that detects formaldehyde using a carbon material has been developed (see, for example, Patent Literature 2). The formaldehyde-detection sensor in Patent Literature 2 includes a reaction unit that contains at least hydroxylamine salts and reacts with formaldehyde to generate an acid, and a response unit that includes an electrode supporting a carbon material of which an electrical resistance value changes by the acid generated in the reaction unit. The sensor is reusable and is capable of constantly monitoring formaldehyde. It is desirable if further applications of the sensor in Patent Literature 2 are developed.

### Citation List

#### Patent Literature

Patent Literature 1: WO 2013/184222
Patent Literature 2: WO 2019/049693

### Disclosure of Invention

#### Technical Problem

From the above, it is an object of the present invention to provide a compact and reusable alkene-detection gas sensor that detects an alkene and a system using the same.

#### Solution to Problem

An alkene-detection gas sensor that detects an alkene in a sample gas according to the present invention

includes: a first reaction unit that contains a palladium catalyst and oxidizes an alkene in a sample gas to convert the alkene into an aldehyde and/or a ketone; a second reaction unit that contains hydroxylamine salts and reacts with the aldehyde and/or ketone converted in the first reaction unit to generate an acid; and a response unit that includes an electrode supporting a semiconductor material of which an electrical resistance value changes by the generated acid, in which the palladium catalyst, the hydroxylamine salts, and the semiconductor material are separated from each other, thereby solving the problem described above.

The alkene may be ethylene.

The palladium catalyst may be a solid catalyst in which metal palladium (Pd) or a palladium ion ($Pd^{2+}$) is supported on an inorganic solid substance.

The inorganic solid substance may be selected from at least one of the group consisting of $V_2O_5$-$TiO_2$, $CeO_2$-$TiO_2$, $V_2O_5$-$CeO_2$, $V_2O_5$-zeolite, $CeO_2$-zeolite, $V_2O_5$-$SiO_2$, $CeO_2$-$SiO_2$, $V_2O_5$-$Al_2O_3$, $CeO_2$-$Al_2O_3$, $Cu_2O$-$TiO_2$, $Cu_2O$-$TiO_2$, CuO-$TiO_2$, $Cu_2O$-zeolite, $Cu_2O$-$SiO_2$, CuO-zeolite, CuO-$SiO_2$, $Cu_2O$-$Al_2O_3$, CuO-$Al_2O_3$, $V_2O_5$-silica alumina, $CeO_2$-silica alumina, $Cu_2O$-silica alumina, CuO-silica alumina, $V_2O_5$-ZnO, $CeO_2$-ZnO, $Cu_2O$-ZnO, CuO-ZnO, $V_2O_5$-$ZrO_2$, $CeO_2$-$ZrO_2$, $Cu_2O$-$ZrO_2$, CuO-$ZrO_2$, $V_2O_5$-$WO_3$, $CeO_2$-$WO_3$, $Cu_2O$-$WO_3$, and CuO-$WO_3$.

The solid catalyst may be represented by the following general formula (1).

$$(Pd+Pd^{2+})_x(V_2O_5)_y(TiO_2)_z \qquad (1)$$

x, y, and z in the formula (1) are numbers satisfying relationships of $0.0001 \leq x \leq 0.1$, $0.001 \leq y \leq 0.5$, $0.40 \leq z \leq 0.998$, and $x+y+z=1$.

The first reaction unit may include a column housing the solid catalyst, and the sample gas may be introduced into the column.

The solid catalyst may be a powder, and the solid catalyst may be supported or encapsulated in a porous material selected from the group consisting of paper, cellulose, a hydrophobic polymer, a hydrophilic polymer, porous glass, glass fiber, a porous carbon material, and a porous oxide.

The hydroxylamine salts may represent a salt obtained by neutralizing hydroxylamine ($NH_2OH$) or a hydroxylamine derivative ($NH_2OR$, where R represents an aromatic, cyclic, or acyclic hydrocarbon compound or a derivative thereof) with an acid selected from the group consisting of hydrogen halide, nitric acid, sulfuric acid, phosphoric acid, boric acid, and trifluoroacetic acid.

The hydroxylamine salts may be encapsulated in a porous filter.

The porous filter may be selected from the group consisting of paper, cellulose, a hydrophobic polymer, a hydrophilic polymer, porous glass, glass fiber, a porous carbon material, and a porous oxide.

A spacer may be further provided between the second reaction unit and the response unit.

A spacer may be further provided between the first reaction unit and the second reaction unit.

The semiconductor material may be a carbon material.

The carbon material may be selected from the group consisting of carbon nanotube, carbon nanohorn, graphene, fullerene, and derivatives thereof.

The carbon nanotube may be a mixture of a semiconductor type single-wall carbon nanotube and a metal type carbon nanotube, and a content ratio of the semiconductor type single-wall carbon nanotube to the metal type carbon nanotube may be larger than 2.

A heating device that heats the first reaction unit may be further provided.

A humidifying device that is located in front of the first reaction unit and humidifies the sample gas may be further provided.

A switching device that switches an introduction destination of the sample gas may be further provided, and the switching device may switch between introducing the sample gas into the first reaction unit, the second reaction unit, and the response unit in this order, introducing the sample gas into the second reaction unit and the response unit in this order without introducing the sample gas into the first reaction unit, and introducing the sample gas into the response unit without introducing the sample gas into the first reaction unit and the second reaction unit.

A third reaction unit that contains at least an oxidizing agent or an oxidation catalyst and reacts with an alcohol to generate an aldehyde and/or a ketone; and a switching device that switches an introduction destination of the sample gas may be further provided, and the switching device may switch between introducing the sample gas into the first reaction unit, the second reaction unit, and the response unit in this order without introducing the sample gas into the third reaction unit, introducing the sample gas into the second reaction unit and the response unit in this order without introducing the sample gas into the first reaction unit and the third reaction unit, introducing the sample gas into the response unit without introducing the sample gas into the first reaction unit, the second reaction unit, and the third reaction unit, and introducing the sample gas into the third reaction unit, the second reaction unit, and the response unit in this order without introducing the sample gas into the first reaction unit.

A third reaction unit that contains at least an oxidizing agent or an oxidation catalyst and reacts with an alcohol to generate an aldehyde and/or a ketone; a first flow path that includes the first reaction unit, the second reaction unit, the response unit connected in series, the sample gas being introduced into the first reaction unit in the first flow path; a second flow path that includes the third reaction unit, the second reaction unit, and the response unit connected in series, the sample gas being introduced into the third reaction unit in the second flow path; a third flow path that includes the second reaction unit and the response unit connected in series, the sample gas being introduced into the second reaction unit in the third flow path; and a fourth flow path that includes the response unit, the sample gas being introduced into the response unit may be further provided. The oxidizing agent or the oxidation catalyst may contain at least $V_2O_5$.

An alkene detection system according to the present invention includes: an alkene-detection gas sensor that detects an alkene in a sample gas; and a detection means, in which the alkene-detection gas sensor is the above-mentioned an alkene-detection gas sensor, and the detection means detects a change in an electrical resistance value from the alkene-detection gas sensor, thereby solving the problem described above.

A control means that controls operations of the alkene-detection gas sensor and the detection means may be further provided.

The alkene-detection gas sensor may further include a switching device that switches an introduction destination of the sample gas.

The alkene-detection gas sensor may further include a third reaction unit that contains at least an oxidizing agent or an oxidation catalyst and reacts with an alcohol to generate an aldehyde and/or a ketone, and the control means may control an operation of the switching device to control a connection state of the first reaction unit, the second reaction unit, the third reaction unit, and the response unit and select at least two or more different connection states, acquire, from the detection means, a change in an electrical resistance value from the response unit measured in each of the selected at least two or more different connection states, and compare the acquired changes in the electrical resistance value with each other to detect an alkene in the sample gas.

The control means may control the switching device to introduce the sample gas into the first reaction unit, the second reaction unit, and the response unit in this order, and acquire, from the detection means, a change in an electrical resistance value from the response unit when the sample gas was introduced into the first reaction unit, control the switching device to introduce the sample gas into the response unit without introducing the sample gas into the first reaction unit and the second reaction unit, and acquire, from the detection means, a change in an electrical resistance value from the response unit when the sample gas was introduced into the response unit, and compare the changes in the electrical resistance value with each other to distinguish a response due to an alkene in the sample gas and a response due to a temperature/humidity change and/or an acid vapor from each other and detect an alkene in the sample gas.

The control means may control the switching device to introduce the sample gas into the first reaction unit, the second reaction unit, and the response unit in this order, and acquire, from the detection means, a change in an electrical resistance value from the response unit when the sample gas was introduced into the first reaction unit, control the switching device to introduce the sample gas into the second reaction unit and the response unit without introducing the sample gas into the first reaction unit, and acquire, from the detection means, a change in an electrical resistance value from the response unit when the sample gas was introduced into the second reaction unit, and compare the changes in the electrical resistance value with each other to distinguish a response due to an alkene in the sample gas and a response due to an aldehyde and/or a ketone in the sample gas from each other and detect an alkene in the sample gas.

The alkene-detection gas sensor may further include a third reaction unit that contains at least an oxidizing agent or an oxidation catalyst and reacts with an alcohol to generate an aldehyde and/or a ketone, and the control means may control the switching device to introduce the sample gas into the first reaction unit, the second reaction unit, and the response unit in this order, and acquire, from the detection means, a change in an electrical resistance value from the response unit when the sample gas was introduced into the first reaction unit, control the switching device to introduce the sample gas into the third reaction unit, the second reaction unit, and the response unit in this order without introducing the sample gas into the first reaction unit, and acquire, from the detection means, a change in an electrical resistance value from the response unit when the sample gas was introduced into the third reaction unit, and compare the changes in the electrical resistance value with each other to distinguish a response due to an alkene in the sample gas and a response due to an alcohol in the sample gas with each other and detect an alkene in the sample gas.

The alkene-detection gas sensor may further include a third reaction unit that contains at least an oxidizing agent or an oxidation catalyst and reacts with an alcohol to generate an aldehyde and/or a ketone, a first flow path that includes the first reaction unit, the second reaction unit, the response unit connected in series, the sample gas being introduced into the first reaction unit in the first flow path, a second flow path that includes the third reaction unit, the second reaction unit, and the response unit connected in series, the sample gas being introduced into the third reaction unit in the second flow path, a third flow path that includes the second reaction unit and the response unit connected in series, the sample gas being introduced into the second reaction unit in the third flow path, and a fourth flow path that includes the response unit, the sample gas being introduced into the response unit. The control means may acquire,

from the detection means, a change in an electrical resistance value from the response unit of the first flow path when the sample gas was introduced into the first flow path, acquire, from the detection means, a change in an electrical resistance value from the response unit of the second flow path when the sample gas was introduced into the second flow path, acquire, from the detection means, a change in an electrical resistance value from the response unit of the third flow path when the sample gas was introduced into the third flow path, acquire, from the detection means, a change in an electrical resistance value from the response unit of the fourth flow path when the sample gas was introduced into the fourth flow path, and compare the changes in the electrical resistance value with each other to distinguish a response due to an alkene in the sample gas, a response due to a temperature/humidity change of the sample gas and/or an acid vapor, a response due to an aldehyde and/or a ketone in the sample gas, and a response due to an alcohol in the sample gas from each other and detect an alkene in the sample gas the sample gas.

Advantageous Effects of Invention

an alkene-detection gas sensor that detects an alkene in a sample gas according to the present invention includes: a first reaction unit that contains a palladium catalyst; a second reaction unit that contains hydroxylamine salts; and a response unit that includes an electrode supporting a semiconductor material of which an electrical resistance value changes by an acid.

Since the first reaction unit contains a palladium catalyst, the first reaction unit is capable of oxidizing an alkene to convert the alkene into an aldehyde and/or a ketone (e.g., capable of converting the alkene into acetaldehyde in the case of the alkene is ethylene). Since the second reaction unit contains hydroxylamine salts, the second reaction unit selectively reacts with an aldehyde and/or a ketone to generate an acid. The generated acid is adsorbed on the semiconductor material contained in the response unit, making it possible to detect an alkene as a change in an electrical resistance value. Here, since the palladium catalyst, the hydroxylamine salts, and the semiconductor material are separated from each other, they do not react with each other and an alkene can be stably detected.

Since the first reaction unit is a catalyst, the first reaction unit is reusable. The hydroxylamine salts contained in the second reaction unit are consumed by a condensation reaction with an aldehyde or a ketone, but is reusable in practice by using an excess amount of hydroxylamine salts compared to the amount of an alkene contained in a sample gas. Since the semiconductor material contained in the response unit adsorbs and desorbs an acid by equilibrium, the semiconductor material is reusable. Further, since it only needs to use an extremely small amount of the catalyst, the hydroxylamine salts, and the semiconductor material, miniaturization is possible.

By combining the alkene-detection gas sensor according to the present invention with a detection means, it is possible to provide a compact and reusable sensor that accurately detects an alkene and a system. In the specification of the subject patent application, the term "compact" means the size and weight that one adult can carry with a margin.

Brief Description of Drawings

[Fig. 1] Fig. 1 is a block diagram showing an alkene-detection gas sensor according to the present invention.
[Fig. 2] Fig. 2 is a schematic diagram showing an exemplary an alkene-detection gas sensor according to the present invention.
[Fig. 3] Fig. 3 is a schematic diagram showing another exemplary an alkene-detection gas sensor according to the present invention.
[Fig. 4] Fig. 4 is a block diagram showing another alkene-detection gas sensor according to the present invention.
[Fig. 5] Fig. 5 is a block diagram showing still another alkene-detection gas sensor according to the present invention.
[Fig. 6] Fig. 6 is a block diagram showing still another alkene-detection gas sensor according to the present invention.
[Fig. 7] Fig. 7 is a block diagram showing an alkene detection system according to the present invention.
[Fig. 8] Fig. 8 is a block diagram showing another alkene detection system according to the present invention.
[Fig. 9A] Fig. 9A is a schematic diagram showing an alkene-detection gas sensor according to an Example 1 (when a clean gas is introduced).
[Fig. 9B] Fig. 9B is a schematic diagram showing the alkene-detection gas sensor according to the Example 1 (when a sample gas is introduced).
[Fig. 10] Fig. 10 is a diagram showing response properties of a sensor according to the Example 1 to ethylene.
[Fig. 11]Fig. 11 is a diagram showing response properties of a sensor according to an Example 2 to 1-hexene.
[Fig. 12] Fig. 12 is a diagram showing response properties of a sensor according to an Example 3 to ethylene.
[Fig. 13] Fig. 13 is a diagram showing response properties of a sensor according to an Example 4 to ethylene.
[Fig. 14] Fig. 14 is a diagram showing response properties of a sensor according to an Example 5 to ethanol.
[Fig. 15] Fig. 15 is a diagram showing response properties of a sensor according to an Example 6 to acetaldehyde.

[Fig. 16] Fig. 16 is a diagram showing response properties of a sensor according to an Example 7 to toluene, acetonitrile, tetrahydrofuran, deuterated chloroform, and methane.
[Fig. 17] Fig. 17 is a diagram showing response properties of a sensor according to an Example 8 to ethylene.
[Fig. 18] Fig. 18 is a diagram showing response properties of a sensor according to an Example 9 to ethylene.
[Fig. 19] Fig. 19 is a schematic diagram showing an alkene-detection gas sensor according to an Example 10.
[Fig. 20] Fig. 20 is a diagram showing the procedure for producing a second reaction unit according to the Example 10.
[Fig. 21] Fig. 21 is a diagram showing response properties of the sensor according to the Example 10 to ethylene.
[Fig. 22] Fig. 22 is a diagram showing response properties of a sensor according to an Example 11 to ethylene.

Mode(s) for Carrying Out the Invention

Hereinafter, embodiments of the present invention will be described with reference to the drawings. Note that similar elements will be denoted by similar reference symbols, and description thereof will be omitted.

(Embodiment 1)

In an embodiment 1, the alkene-detection gas sensor according to the present invention and the operation thereof will be described.
Fig. 1 is a block diagram showing the alkene-detection gas sensor according to the present invention. an alkene-detection gas sensor 100 includes a first reaction unit 110 that contains a palladium catalyst and oxidizes an alkene in a sample gas to convert the alkene into an aldehyde and/or a ketone, a second reaction unit 120 that contains hydroxylamine salts and reacts with the aldehyde and/or ketone converted in the first reaction unit to generate an acid, and a response unit 130 that includes an electrode supporting a semiconductor material of which an electrical resistance value changes by the generated acid, and the palladium catalyst, the hydroxylamine salts, and the semiconductor material are separated from each other. In the specification of the subject patent application, the term "separated from each other" represents a physically non-mixed state. One second reaction unit 120 and one response unit 130 are shown in Fig. 1, but the number thereof may be two or more.
Each component will be described in detail.
As the palladium catalyst, a homogeneous (solution) and heterogeneous (solid) palladium catalyst can be adopted, but a heterogeneous catalyst (solid phase catalyst) in which a palladium ion($Pd^{2+}$) or metal palladium (Pd(0)) is supported on an inorganic solid substance is excellent in handling as a sensor and is favorable in improving the contact efficiency between the palladium catalyst and an alkene.
The inorganic solid substance supporting a palladium ion($Pd^{2+}$) or metal palladium is, for example, an inorganic solid material such as activated carbon, zeolite, titanium dioxide, and silica gel. Further, favorably, copper chloride ($CuCl_2$, CuCl) or vanadium oxide ($V_2O_5$) is added thereto. As a result, oxidation (generally, this catalyst rection is known as the Wacker oxidation reaction) of an alkene into an aldehyde and/or a ketone is activated and the stability can be improved.
The inorganic solid substance is favorably selected from the group consisting of $V_2O_5$-$TiO_2$, $CeO_2$-$TiO_2$, $V_2O_5$-$CeO_2$, $V_2O_5$-zeolite, $CeO_2$-zeolite, $V_2O_5$-$SiO_2$, $CeO_2$-$SiO_2$, $V_2O_5$-$Al_2O_3$, $CeO_2$-$Al_2O_3$, $Cu_2O$-$TiO_2$, $Cu_2O$-$TiO_2$, CuO-$TiO_2$, $Cu_2O$-zeolite, $Cu_2O$-$SiO_2$, CuO-zeolite, CuO-$SiO_2$, $Cu_2O$-$Al_2O_3$, CuO-$Al_2O_3$, $V_2O_5$-silica alumina, $CeO_2$-silica alumina, $Cu_2O$-silica alumina, CuO-silica alumina, $V_2O_5$-ZnO, $CeO_2$-ZnO, $Cu_2O$-ZnO, CuO-ZnO, $V_2O_5$-$ZrO_2$, $CeO_2$-$ZrO_2$, $Cu_2O$-$ZrO_2$, CuO-$ZrO_2$, $V_2O_5$-$WO_3$, $CeO_2$-$WO_3$, $Cu_2O$-$WO_3$, and CuO-$WO_3$. Here, the notation "$V_2O_5$-$TiO_2$" means that $V_2O_5$ is added to $TiO_2$, and the same applies to the others.
The solid catalyst is favorably represented by $(Pd+Pd^{2+})_x(V_2O_5)_y(TiO_2)_z$. x, y, and z in the formula satisfy the relationships of $0.0001 \leq x \leq 0.1$, $0.001 \leq y \leq 0.5$, $0.40 \leq z \leq 0.998$, and $x+y+z=1$. By using such a solid catalyst, the above-mentioned Wacker reaction is promoted.
The solid catalyst is favorably a powder and may be supported or encapsulated in a porous material. This facilitates the separation of the palladium catalyst, the hydroxylamine salts, and the semiconductor material.
Such a porous material is favorably selected from the group consisting of paper, cellulose, a hydrophobic polymer, a hydrophilic polymer, porous glass, glass fiber, a porous carbon material, and a porous oxide. With these materials, a sample gas is introduced and the Wacker reaction is not interfered.
Favorably, the porous material has a specific surface area in the range of 10 $m^2$/g or more and 5000 $m^2$/g or less, has a pore diameter in the range of 10 nm or more and 100 $\mu$m or less, and a pore volume of 0.05 $cm^3$/g or more and 0.90 $cm^3$/g or less. As a result, a solid catalyst that is necessary for a reaction and does not require future replacement can be supported.
The first reaction unit 110 may include a column filled with a palladium catalyst or a solid catalyst. As a result, it is possible to efficiently bring an alkene contained in a sample gas and the palladium catalyst into contact with each other. Since the sample gas is introduced into the column, the contact time with the palladium catalyst becomes longer

by slowing down the flow rate of the sample gas passing through the column, and the conversion rate from an alkene into an aldehyde/a ketone can be improved.

The first reaction unit 110 may further include a heating device. As a result, the activity of the Wacker reaction catalyst can increase. As the heating device, an arbitrary heating device capable of heating the palladium catalyst in the temperature range of 25°C or more and 100°C or less can be adopted.

The first reaction unit 110 may further include a device that adds oxygen ($O_2$) and a humidifying device that adds water vapor ($H_2O$) in the front stage. As a result, the activity of the Wacker reaction catalyst can increase.

The hydroxylamine salts in the second reaction unit 120 can be obtained by neutralizing hydroxylamine ($NH_2OH$) with a volatile acid. Examples thereof include a neutralizing salt selected from the group consisting of a halide, a nitrate, and a trifluoroacetate. In the case where the acid generated in the second reaction unit 120 is volatile, the acid is capable of reaching the separated response unit 130 by diffusion.

Note that those obtained by mixing a neutralizing salt including a volatile acid such as NaCl in a neutralizing salt including a non-volatile acid such as a sulfate, a phosphate, and a borate are also included in the above-mentioned salt obtained by being neutralized by a volatile acid (e.g., a halide, a nitrate, and a trifluoroacetate).

These hydroxylamine salts are readily available or can be easily synthesized. Among them, those that generate a highly volatile strong acid when reacting with an aldehyde or a ketone are favorable because the semiconductor material contained in the response unit 130 is strongly hole-doped, which leads to highly sensitive detection of an alkene. Examples thereof include a halide of hydroxylamine ($NH_2OH{\cdot}HCl$, $NH_2OH{\cdot}HBr$) and a trifluoroacetate ($NH_2OH{\cdot}CF_3COOH$).

Alternatively, the hydroxylamine salts represent a neutralizing salt of an organic compound selected from the group consisting of a halide, a nitrate, and a trifluoroacetate of a hydroxyamine derivative $NH_2OR$ (R represents an aromatic, cyclic, or acyclic hydrocarbon compound, or a derivative thereof). Examples thereof include methylated or acetylated hydroxylamine salts. Among them, R represents a halide ($NH_2OR{\cdot}HCl$, $NH_2OR{\cdot}HBr$), a trifluoroacetate ($NH_2OR{\cdot}CF_3COOH$), or the like containing an aromatic benzene ring or nitrobenzene, and O-benzylhydroxylamine hydrochloride and the like are exemplified. Note that in the present specification, the "derivative" represents a compound or the like modified to such an extent that the original structure and the properties do not significantly change even if introduction of a functional group, oxidation, reduction, substitution of an atom, or the like.

Alternatively, the hydroxylamine salts may represent a solid material $NH_2OQ$ (Q represents a polymer, polymer beads, silica gel, or the like) in which hydroxylamine is supported on the surface of a polymer or an inorganic compound, which has been neutralized by treatment with a halogen acid, nitric acid, or trifluoroacetic acid.

Instead of the hydroxylamine salts, amine salts or hydrazine salts known to cause a condensation reaction with a carbonyl compound, and a derivative thereof may be used.

In the case where the hydroxylamine salts are in a crystalline or powder state, the hydroxylamine salts may be encapsulated in a porous filter. This simplifies the handling of the second reaction unit 120.

Such a porous filter favorably has air permeability and only needs to be formed of a material that is not reactive with hydroxylamine salts and has pores capable of supporting hydroxylamine salts. For example, the porous filter is formed of a material selected from the group consisting of paper typified by filter paper, a hydrophobic polymer, a hydrophilic polymer, a polymer filter, porous glass, a porous carbon material, and a porous oxide. These are porous filters for which commercial products are readily available. For the purpose of preventing wetting with water, a porous filter formed of a hydrophobic polymer such as polytetrafluoroethylene (PTFE) is favorable.

The second reaction unit 120 may include a column filled with hydroxylamine salts. As a result, it is possible to efficiently bring the carbonyl compound (an aldehyde or a ketone) generated in the first reaction unit 110 and hydroxylamine salts into contact with each other. Since the gas containing the generated carbonyl compound is introduced into the column, the contact time with the hydroxylamine salts becomes longer by slowing down the flow rate of the gas passing through the column, and the conversion rate from an aldehyde/a ketone into an acid can be improved.

The semiconductor material in the response unit 130 is not particularly limited as long as it is a semiconductor material of which an electrical resistance value changes by adsorption of an acid. Examples of such a material include a carbon material, a conductive polymer, and an inorganic semiconductor. Whether the electrical resistance value increases or decreases by adsorption of an acid depends on the properties of the semiconductor material, but those of any property can be used. In many p-type semiconductors of which an electrical resistance value decreases by hole doping, the electrical resistance value decreases by adsorption of an acid. The carbon nanotube used in the present specification is a p-type semiconductor and is known that the electrical resistance value decreases by contact with an acid.

The carbon material is, for example, a material selected from the group consisting of carbon nanotube, carbon nanohorn, graphene, fullerene, and derivatives thereof. It is known that electrical resistance values of these change by adsorption of an acid. Among them, carbon nanotube is favorable because it is readily available and has excellent electrical physical properties and stability. The derivatives represent those having a functional group such as an amine and carboxylic acid on the surface, or those having a surface coated with a dispersant or the like.

Further, carbon nanotube can be grouped into single-wall, double-wall, and multi-wall carbon nanotubes in

accordance with the number of overlapping layers of graphene, but any of them can be adopted in the present invention. Among them, some of the single-wall carbon nanotubes (SWCNTs) are semiconducting and favorable because an electrical resistance value easily changes by an acid. Further, the surface of SWCNT is a hydrophobic and stable graphene sheet and is favorable because a response to a humidity change is small and it is not easily denatured by an acid.

Whether the SWCNT is a semiconductor or a metal body is determined by the chirality of the way of winding the graphene sheet, a synthesized SWCNT is a mixture containing a semiconductor and a metal body in the ratio of 2:1. For the purpose of increasing the response sensitivity of the SWCNT to a vapor of an acid, i.e., the amount of change in an electrical resistance value, the semiconductor SWCNT may be separated and purified, and the semiconductor SWCNT may be used as a semiconductor material. As the method of separating and purifying the semiconductor, the column separation method described in Patent Literature 2, or the like can be adopted. The content ratio of the semiconductor single-wall carbon to a metal type carbon nanotube may be larger than 2.

Further, usually, by dispersing SWCNTs forming a bundle structure by strong $\pi$-$\pi$ interaction one by one and then applying then on an electrode, the surface area of the SWCNT capable of coming into contact with an acid vapor increases, and thus, the response sensitivity to an acid vapor increases, whereby it is possible to increase the detection sensitivity of an aldehyde. As the method of dispersing the SWCNT, dispersion treatment with a dispersant in combination with ultrasonic treatment in a solvent can be adopted. As the dispersant, a $\pi$-conjugated polymer such as polyfluorene and an amphipathic surfactant such as sodium dodecyl sulfate can be adopted in addition to the supramolecular polymer described in Patent Literature 2. As the solvent, an organic solvent and water can be adopted. Among the organic solvents, orthodichlorobenzene is known to have dispersibility of SWCNT and is favorable.

The conductive polymer used as a semiconductor material is, for example, a conductive polymer selected from the group consisting of polythiophene, polyaniline, polypyrrole, polyacetylene, polyparaphenylenevinylene, polyparaphenylene, and derivatives thereof. Since electrical resistance values of these conductive polymers change by being in contact with a vapor of an acid, they can be adopted as a semiconductor material in the response unit 130.

For example, in the case of polythiophene that is a p-type semiconductor, the conductivity increases by being hole-doped by an acid as in PEDOT (poly(3,4-ethylenedioxythiophene)-PSS (poly(4-styrene sulfonic acid)) that has been widely put to practical use. Further, a conductive polymer containing a basic functional group such as polyaniline and polypyrrole is more favorable because it has a high affinity with acid molecules and an electrical resistance value changes as the band gap changes due to adsorption of acid molecules.

As the inorganic semiconductor, for example, an inorganic oxide semiconductor having a proven track record as a gas sensor such as $SnO_2$ (tin oxide), $In_2O_3$ (indium oxide), ZnO (zinc oxide), and $Fe_2O_3$ (iron oxide) can be adopted.

The semiconductor material favorably has a large specific surface area by overlapping with each other while having an internal space so that an acid is easily adsorbed thereon, and may form a porous body or a network structure (network). The semiconductor material is desirably a thin film in order to improve the detection sensitivity.

The semiconductor material is supported on an electrode formed of an electrode material normally used, and the electrode is formed of a material selected from the group consisting of a conductive carbon material such as Au, Pt, Ag, alloys thereof, and glassy carbon. Examples of the shape of the electrode include a comb-shaped electrode.

The operation principle of a sensor 100 according to the present invention will be described.

The first reaction unit 110 containing a palladium catalyst oxidizes an alkene contained in a sample gas to an aldehyde and/or a ketone. Examples of the substrate involved in the oxidation of an alkene include oxygen ($O_2$) and water ($H_2O$). In the case where the above-mentioned substrate is oxygen and water, it is desirable because they can be readily available from atmosphere components.

In the second reaction unit 120, hydroxylamine salts cause a condensation reaction with the carbonyl compound (an aldehyde or a ketone) generated in the first reaction unit 110 to generate a vapor of an acid. Here, for the sake of simplicity, the case of using acetaldehyde as a carbonyl compound and hydroxylamine hydrochloride as hydroxylamine salts is shown in the following formula. Even in the case of a combination of another carbonyl compound and hydroxyamine salts, an acid is generated by a similar condensation reaction.

$$CH_3CHO+NH_2OH\cdot HCl\rightarrow CH_3CH{=}NOH+H_2O+HCl$$

The electrical resistance value of the semiconductor material contained in the response unit 130 changes by the acid generated in the second reaction unit 120. As a result, it is possible to determine that an alkene is contained in a sample gas. In detail, in the case where the semiconductor material is of a p-type, holes are injected by adsorption of an acid and the electrical resistance value often decreases. The gas to be introduced into the alkene-detection gas sensor 100 is switched from a clean gas containing no an alkene to a sample gas for which an alkene is desired to be detected, an electrical resistance value $R_t$ (or current value $I_t$) of the response unit 130 after a lapse of a certain period of time is measured, and an alkene is detected on the basis of the relative amount of change in an electrical resistance value represented by the following formula. Further, the concentration of an alkene may be converted from the correlation (calibration curve prepared separately) between the above-mentioned relative amount of change and the concentration

of an alkene. Ro represents an electrical resistance value of the response unit 130 under a clean gas atmosphere.

$$\text{Relative amount of change in an electrical resistance value (\%)} = (R_t - R_0)/R_0 \times 100$$

After the measurement is completed, by switching the gas to be introduced into the sensor 100 to a clean gas, acid molecules adsorbed on the semiconductor material are desorbed at equilibrium and thus, the electrical resistance value recovers to the state before the start of the measurement after a certain period of time, thereby making it possible to perform repeated measurement.

The concentration of an aldehyde and/or a ketone generated in the first reaction unit 110 correlates with the concentration of the supplied an alkene, and the concentration of an acid generated in the second reaction unit 120 correlates with the concentration of the supplied aldehyde and/or ketone. Further, the change in an electrical resistance value that occurs in the response unit 130 correlates with the concentration of the supplied acid. Therefore, by adopting a mechanism in which the reaction unit 110, the reaction unit 120, and the response unit 130 are separated from each other can connected to each other, it is possible to quantify the concentration of an alkene using the calibration curve prepared in advance.

Fig. 2 is a schematic diagram showing an exemplary alkene-detection gas sensor according to the present invention.

an alkene-detection gas sensor 200 shown in Fig. 2 includes the first reaction unit 110 that includes a palladium catalyst 140, the second reaction unit 120 that includes hydroxylamine hydrochloride 150, and the response unit 130 that includes an electrode 170 supporting a semiconductor material 180. The electrode 170 is disposed on a substrate 160, and the palladium catalyst 140, the hydroxylamine salts 150, and the semiconductor material 180 are separated from each other. Columns are filled with the palladium catalyst 140 and the hydroxylamine salts 150, and a sample gas is introduced into the column of the first reaction unit 110.

In the first reaction unit 110, an alkene contained in a sample gas is converted into acetaldehyde and/or a ketone by being in contact with the palladium catalyst 140, and is supplied to the second reaction unit. Subsequently, in the second reaction unit, the acetaldehyde and/or ketone reacts with the hydroxylamine salts 150 to generate a vapor of an acid, and the vapor of an acid is supplied to the response unit 130. In the response unit 130, a constant voltage is applied to the electrode 170 and a current value flowing through the semiconductor material 180 is measured by an ammeter. When the conductivity of the semiconductor material 180 changes by the vapor of an acid, the ammeter detects the change in an electrical resistance value as an electrical signal, and it is possible to determine that a sample gas contains an alkene.

Such an alkene-detection gas sensor 200 is produced, for example, as follows. First, the substrate 160 including the electrode 170 is prepared. The semiconductor material 180 is dissolved or dispersed in a solvent. The solvent is not particularly limited as long as it is volatile, but is, for example, o-dichlorobenzene. Subsequently, this solution/dispersion is drop cast onto the electrode 170. After drying the solvent, the response unit 130 is obtained.

A palladium catalyst is packed in a glass tube and incorporating it into the gas flow path, thereby constructing the first reaction unit 110. Hydroxylamine salts are packed in a glass tube and incorporating them into the gas flow path in the subsequent stage of the first reaction unit 110, thereby constructing the second reaction unit 120. By installing the response unit 130 in the subsequent stage of the second reaction unit 120, a sensor 200 according to the present invention is obtained.

Fig. 3 is a schematic diagram showing another exemplary alkene-detection gas sensor according to the present invention.

Alkene-detection gas sensor 300 in Fig. 3 includes the first reaction unit 110 that includes the palladium catalyst 140, the second reaction unit 120 that includes the hydroxylamine hydrochloride 150, and the response unit 130 that includes the electrode 170 supporting the semiconductor material 180. The electrode 170 is disposed on the substrate 160, and the palladium catalyst 140, the hydroxylamine salts 150, and the semiconductor material 180 are separated from each other. The palladium catalyst 140 and the hydroxylamine salts 150 are supported on a porous filter, and a sample gas is introduced into an entire sensor 300.

In the alkene-detection gas sensor 300, an alkene is in contact with the palladium catalyst 140 to generate an aldehyde or a ketone, the generated aldehyde or ketone is in contact with the hydroxylamine hydrochloride 150 by diffusion to generate an acid, and the generated acid is in contact with the semiconductor material 180 by diffusion, which is detected as a change in an electrical resistance value in the response unit 130. Note that the alkene that has directly come into contact with the hydroxylamine hydrochloride 150 and the semiconductor material 180 does not affect the response unit. Such an alkene-detection gas sensor 300 is produced, for example, as follows.

A palladium catalyst and hydroxylamine salts are added to solvents such as methanol, and the dispersions or

solutions are drop cast onto porous materials. By removing the solvents by drying, the first reaction unit 110 and the second reaction unit 120 are obtained. A spacer 190 is installed on the response unit 130 obtained in the same way as that in the sensor 200, the reaction unit 120 and the spacer 190 are installed thereon, and the first reaction unit 110 is installed thereon, whereby the first reaction unit 110, the second reaction unit 120, and the response unit 130 are disposed to be separated from each other. The spacer 190 has a size of, for example, 0.2 mm, and the material thereof is not limited.

Although not shown, a first reaction unit/a second reaction unit including a column and a second reaction unit/a first reaction unit including a porous filter or a porous material may be combined with each other.

There is a possibility that the response unit 130 can elicit a response similar to that of an alkene, by a chemical substance other than an alkene. An acid gas, an alcohol vapor, an aldehyde vapor, and a ketone vapor are assumed as chemical substances that are easily configures with an alkene. Hereinafter, for the sake of simplicity, an alcohol vapor, an aldehyde vapor, and a ketone vapor will be referred to simply as an alcohol, an aldehyde, and a ketone in some cases.

For example, an acid gas and a temperature/humidity change directly act on the response unit 130 to change the conductivity of the semiconductor material, and thus is easily confused with a vapor of an acid generated from an alkene through the first reaction unit 110 and the second reaction unit 120.

Since the first reaction unit 110 is an oxidation catalyst, there is a possibility that an alcohol vapor is oxidized to generate an aldehyde and/or a ketone depending on the activity thereof. The aldehyde and/or ketone generated from an alcohol generates a vapor of an acid in the second reaction unit 120. For this reason, a vapor of an acid derived from an alcohol is easily confused with a vapor of an acid derived from an alkene.

Vapors of an aldehyde and a ketone directly act on the second reaction unit 120 to generate a vapor of an acid. For this reason, the vapor of an acid derived from an aldehyde and a ketone is easily confused with a vapor of an acid generated from an alkene.

An alkene-detection gas sensor capable of clearly distinguishing a response due to an alkene in a sample gas and a response due to an acid gas, an alcohol, an aldehyde, a ketone, and a temperature/humidity change with each other will be described with reference to Fig. 4 to Fig. 6.

Fig. 4 is a block diagram showing another an alkene-detection gas sensor according to the present invention.

An alkene-detection gas sensor 400 is similar to the alkene-detection gas sensor 100 except that it includes a switching device 410 of a flow path of a sample gas in addition to the alkene-detection gas sensor 100 shown in Fig. 1.

The switching device 410 switches between introducing a sample gas into the first reaction unit 110, the second reaction unit 120, and the response unit 130 in this order, introducing a sample gas into the second reaction unit 120 and the response unit 130 in this order without introducing the sample gas into the first reaction unit 110, and introducing a sample gas into the response unit 130 without introducing the sample gas into the first reaction unit 110 and the second reaction unit 120.

In the case where the switching device 410 has switched to introduce a sample gas into the first reaction unit 110, the second reaction unit 120, and the response unit 130 in this order, the operation similar to that of the alkene-detection gas sensor 100 shown in Fig. 1 is performed.

In the case where the switching device 410 has switched to introduce a sample gas into the second reaction unit 120 and the response unit 130 bypassing the first reaction unit 110, no response is observed in the response unit 130 because an alkene itself does not generate an acid gas in the second reaction unit 120 even if an alkene is present in the sample gas. Meanwhile, if an aldehyde or a ketone is present in a sample gas, a response is observed in the response unit 130 because a vapor of an acid is generated when the sample gas reaches the second reaction unit 120 even in the case of bypassing the first reaction unit 110.

Therefore, by comparing a response (electrical resistance value) of the response unit 130 in the case where a sample gas has been applied to the first reaction unit 110 and a response of (electrical resistance value) of the response unit 130 in the case where a sample gas has been applied to the second reaction unit 120 bypassing the first reaction unit 110 with each other, it is possible to clearly distinguish a response due to an alkene and a response due to an aldehyde and a ketone from each other and accurately detect an alkene gas.

In the case where the switching device 410 has switched to introduce a sample gas into the response unit 130 bypassing the first reaction unit 110 and the second reaction unit 120, no response is observed in the response unit 130 because an alkene, an aldehyde, and a ketone contained in a sample gas do not change the conductivity of the semiconductor in the response unit 130. Meanwhile, a vapor of an acid in a sample gas or a temperature/humidity change of a sample gas directly acts on the response unit 130 to show a response even in the case of bypassing the first reaction unit 110 and the second reaction unit 120.

Therefore, by comparing a response of the response unit 130 in the case where a sample gas has been applied to the first reaction unit 110 and the second reaction unit 120 and a response of the response unit 130 in the case of bypassing the first reaction unit 110 and the second reaction unit 120 with each other, it is possible to distinguish a response due to an alkene and a response due to a temperature/humidity change and/or an acid vapor from each other.

Fig. 5 is a block diagram showing still another an alkene-detection gas sensor according to the present invention.

An alkene-detection gas sensor 500 in Fig. 5 further includes a third reaction unit 510 that reacts with an alcohol to generate an aldehyde and/or a ketone, in addition to the alkene-detection gas sensor 400 shown in Fig. 4. The third reaction unit 510 includes an oxidizing agent or an oxidation catalyst having an ability to oxidize an alcohol to an aldehyde and/or a ketone, and does not have an ability to oxidize an alkene to an aldehyde and/or a ketone. In Fig. 5, the first reaction unit 110 and the third reaction unit 510 are disposed in parallel.

Such an oxidizing agent or an oxidation catalyst is titanium dioxide or the like supporting pyridinium chlorochromate, pyridinium dichromate, vanadium oxide ($V_2O_5$), or the like, but favorably contains at least $V_2O_5$.

The switching device 410 switches between introducing a sample gas into the first reaction unit 110, the second reaction unit 120, and the response unit 130 in this order without introducing a sample gas into the third reaction unit 510, introducing a sample gas into the second reaction unit 120 and the response unit 130 in this order without introducing a sample gas into the first reaction unit 110 and the third reaction unit 510, introducing a sample gas into the response unit 130 without introducing a sample gas into the first reaction unit 110, the second reaction unit 120, and the third reaction unit 510, and introducing a sample gas into the third reaction unit 510, the second reaction unit 120, and the response unit 130 in this order without introducing a sample gas into the first reaction unit 110.

In the case where the switching device 410 has switched to introduce a sample gas into the third reaction unit 510, the second reaction unit 120, and the response unit 130 bypassing the first reaction unit 110, a response is observed in the response unit 130 when an alcohol is present in the sample gas.

Therefore, by comparing a response of the response unit 130 in the case where a sample gas has been introduced into the first reaction unit 110, the second reaction unit 120, and the response unit 130 without introducing a sample gas into the third reaction unit 510 and a response of the response unit 130 in the case where a sample gas has been introduced into the third reaction unit 510, the second reaction unit 120, and the response unit 130 without introducing a sample gas into the first reaction unit 110 with each other, it is possible to distinguish a response due to an alkene and a response due to an alcohol from each other.

It goes without saying that by switching an introduction destination of a sample gas by the switching device 410, it is possible to distinguish a response due to an alkene, a response due to an aldehyde and/or a ketone, and a response due to a temperature/humidity change and/or an acid vapor from each other, similarly to the alkene-detection gas sensor 400 in Fig. 4.

Fig. 6 is a block diagram showing still another an alkene-detection gas sensor according to the present invention.

An alkene-detection gas sensor 600 in Fig. 6 includes the first reaction unit 110, two or more second reaction units 120, and two or more response units 130 and further includes the third reaction unit 510 that reacts with an alcohol to generate an aldehyde and/or a ketone.

The alkene-detection gas sensor 600 includes a plurality of second reaction units (120-1 to 120-3), a plurality of response units (130-1 to 130-4), and first to fourth flow paths 610 to 640 through which a sample gas flows.

The first flow path 610 includes the first reaction unit 110, the second reaction unit 120-1, and the response unit 130-1 connected in series, and a sample gas is introduced into the first reaction unit 110.

The second flow path 620 includes the third reaction unit 510, the second reaction unit 120-2, and the response unit 130-2 connected in series, and a sample gas is introduced into the third reaction unit 510.

The third flow path 630 includes the second reaction unit 120-3 and the response unit 130-3 connected in series, and a sample gas is introduced into the second reaction unit 120-3.

The fourth flow path 640 includes the response unit 130-4 and a sample gas is introduced into the response unit 130-4.

The first flow path 610 of the alkene-detection gas sensor 600 responds to an alkene in a sample gas. However, as described above, there is a possibility that the response detected in the response unit 130-1 of the first flow path 610 is a false response derived from an alcohol, an aldehyde, a ketone, an acid vapor, or a temperature/humidity change.

The second flow path 620 responds to an alcohol, an aldehyde, a ketone, an acid vapor, or a temperature/humidity change of a sample gas. However, the second flow path 620 does not respond to an alkene. The third flow path 630 responds to an aldehyde, a ketone, an acid vapor, or a temperature/humidity change. However, the third flow path 630 does not respond to an alkene and an alcohol. The fourth flow path 640 responds to a temperature/humidity change or an acid vapor. However, the fourth flow path 640 does not responds to an alkene, an alcohol, an aldehyde, and a ketone. For the sake of simplicity, the detection is listed below.

(Table 1)

Table 1: List of presence or absence of response of gas type, etc. in 1st stage to 4th stage

| Gas type, etc. | 1st stage | 2nd stage | 3rd stage | 4th stage |
|---|---|---|---|---|
| Alkene | ○ | × | × | × |
| Alcohol | △ | ○ | × | × |

(continued)

| Gas type, etc. | 1st stage | 2nd stage | 3rd stage | 4th stage |
|---|---|---|---|---|
| Aldehyde | △ | ○ | ○ | × |
| Ketone | △ | ○ | ○ | × |
| acid | △ | ○ | ○ | ○ |
| Temperature/humidity change | △ | ○ | ○ | ○ |
| ○ : Response, × : No response, △ : False response | | | | |

By analyzing the responses simultaneously collected from the plurality of response units 130 in this way, it is possible to clearly distinguish a response due to an alkene in a sample gas and a response due to another chemical substance that is easily confused with an alkene from each other.

Although three second reaction units 120-1 to 120-3 and four response units 130-1 to 130-4 have been used in Fig. 6, the number of the second reaction units 120 and the number of the response units 130 are merely examples and are not limited thereto. More or less second reaction units 120 and response units 130 may be provided.

The response unit 130 has been described as using an electrode including the semiconductor material 130 of which an electrical resistance value changes by an acid, but is not limited thereto. As the response unit 130, an arbitrary element that responds to an acid and an electrical signal changes can be adopted. Examples thereof include an element of which capacitance changes, an element of which a resonance frequency changes (e.g., quartz crystal microbalance), and an element in which mechanical displacement occurs (e.g., cantilever). By combining these, it is possible to detect an alkene.

(Embodiment 2)

In an embodiment 2, a system using the alkene-detection gas sensor according to the present invention described in the embodiment 1 will be described.

Fig. 7 is a block diagram showing an alkene detection system according to the present invention.
an alkene detection system 700 according to the present invention (hereinafter, referred to simply as the system according to the present invention in some cases) includes at least the sensors 100 to 300 (Fig. 1 to Fig. 3) according to the present invention and a detection means 710 that detects a change in an electrical resistance value from the sensors 100 to 300. In Fig. 7, the system 700 according to the present invention is connected to a power source 720. The power source 720 can be a fixed power source, a battery, or the like.

The detection means 710 is not particularly limited as long as it is capable of detecting a change in an electrical resistance value, but is, for example, an ammeter or a voltmeter. With an ammeter, if the voltage is known, it is possible to detect a change in an electrical resistance value of the response unit 130 by measuring the magnitude of a current. The voltage may be a direct current or an alternating current.

The operation principle of the system 700 according to the present invention will be described.

When a sample gas is introduced into the sensors 100 to 300, in the case where the sample gas contains an alkene, an electrical resistance value changes in the response unit 130 of each of the sensors 100 to 300 as described above. The detection means 710 detects the change in an electrical resistance value that has occurred in the response unit 130. In this way, it is possible to detect that an alkene is present in the sample gas.

Alternatively, the system may include a control means (not shown) storing data of a false response based on temperature and humidity in a database, and the control means may compare a change in an electrical resistance value of the response unit 130 in each of the sensors 100 to 300 according to the present invention, which has been detected by the detection means 710, and the data stored in the database of the control means with each other to distinguish a positive response and a false response from each other and correct the concentration.

Fig. 8 is a block diagram showing another an alkene detection system according to the present invention.
an alkene detection system 800 according to the present invention includes at least the sensors 400 to 600 (Fig. 4 to Fig. 6) according to the present invention, the detection means 710 that detects a change in an electrical resistance value from the sensors 400 to 600, and a control means 810 that controls the operations of the sensors 400 to 600 and the detection means 710. Also in this case, the control means 810 can include a memory or the like that stores the database as described above. The control means 810 may include a central processing unit (CPU), a memory, and, if necessary, a communication means, and a personal computer can be used.

The operation principle of the system 800 according to the present invention will be described.

Here, a case where the system 800 uses the sensors 500 and 600 including the third reaction units 510 (Fig.

5 and Fig. 6) that react with an alcohol to generate an aldehyde and/or a ketone will be described.

The control means 810 controls the operation of the switching device 410 (Fig. 5) to control the connection state of the first reaction unit 110 (Fig. 5), the second reaction unit 120 (Fig. 5), the third reaction unit 510 (Fig. 5), and the response unit 130 (Fig. 5), select at least two or more different connection states, and acquire, from the detection means 710, a change in an electrical resistance value from the response unit 130 (Fig. 5) measured in each of the selected at least two or more different connection states. The control means 810 compares the acquired changes in the electrical resistance value with each other to detect an alkene in the sample gas.

Examples of the different connection states include a first connection state in which the first reaction unit 110, the second reaction unit 120, and the response unit 130 are connected in this order, a second connection state in which the third reaction unit 510, the second reaction unit 120, and the response unit 130 are connected in this order, a third connection state in which the second reaction unit 120 and the response unit 130 are connected in this order, and a fourth connection state in which only the response unit 130 is connected.

In the at least two or more different connection states, the first connection state is always selected and at least one or more of the second to fourth connection states are selected.

Description will be made in more detail.

The operation principle in the case where the system 800 according to the present invention includes the sensor 400 will be described.

The control means 810 controls the switching device 410 (Fig. 4) of the sensor 400 and the detection means 710 as follows.

(Control 1) The control means 810 controls the switching device 410 (Fig. 4) to introduce a sample gas into the first reaction unit 110 (Fig. 4), the second reaction unit 120 (Fig. 4), and the response unit 130 (Fig. 4) in this order, and acquires, from the detection means 710, a change in an electrical resistance value from the response unit 130 at this time.

(Control 2) The control means 810 controls the switching device 410 to introduce a sample gas into the second reaction unit 120 and the response unit 130 without introducing a sample gas into the first reaction unit 110, and acquires, from the detection means 710, a change in an electrical resistance value from the response unit 130 at this time. Note that the acquired data may be stored in an internal memory (not shown) or the like.

(Control 3) The control means 810 controls the switching device 410 to introduce a sample gas into the response unit 130 without introducing a sample gas into the first reaction unit 110 and the second reaction unit 120, and acquires, from the detection means 710, a change in an electrical resistance value from the response unit 130 at this time.

The control means 810 compares a change in an electrical resistance value obtained when executing the Control 1 and a change in an electrical resistance value obtained when executing the Control 2 with each other to distinguish a response (positive response) due to an alkene in the sample gas and a response (false response) due to an aldehyde and/or a ketone in the sample gas from each other and detect an alkene in the sample gas.

The control means 810 is capable of determining, in the case where a change in an electrical resistance value obtained when executing the Control 1 is significantly large and a change in an electrical resistance value obtained when executing the Control 2 is substantially zero (corresponding to the error level of the detection means 710), that an alkene is present in a sample gas, and detects, in the case where any change in an electrical resistance value is significantly large, that it is a false response due to the presence of an aldehyde and/or a ketone in the sample gas.

The control means 810 compares a change in an electrical resistance value obtained when executing the Control 1 and a change in an electrical resistance value obtained when executing the Control 3 with each other to detect a response (positive response) due to an alkene in a sample gas and a response (false response) due to a temperature/humidity change and/or an acid vapor from each other and detect an alkene in the sample gas.

The control means 810 is capable of determining, in the case where a change in an electrical resistance value obtained when executing the Control 1 is significantly large and a change in an electrical resistance value obtained when executing the Control 3 is substantially zero (corresponding to the error level of the detection means 710), that an alkene is present in a sample gas, and determines, in the case where any change in an electrical resistance value is significantly large, that it is a false response due to an acid vapor present in a sample gas and/or a temperature/humidity change of a sample gas.

The control means 810 may execute only the Control 1 and the Control 2, only the Control 1 and the Control 3, and all the of Control 1 to the Control 3.

The operation principle in the case where the system 800 according to the present invention includes the sensor 500 will be described.

The control means 810 controls the switching device 410 (Fig. 5) of the sensor 500 and the detection means 710 as follows.

(Control 1) The control means 810 controls the switching device 410 to introduce a sample gas into the first reaction unit 110 (Fig. 5), the second reaction unit 120 (Fig. 5), and the response unit 130 (Fig. 5) in this order and acquire, from the detection means 710, a change in an electrical resistance value from the response unit 130 at this time.

(Control 4) The control means 810 controls the switching device 410 to introduce a sample gas into the third reaction unit 510, the second reaction unit 120, and the response unit 130 in this order without introducing a sample gas into the first reaction unit 110 and acquire, from the detection means 710, a change in an electrical resistance value from the response unit 130 at this time.

The control means 810 compares a change in an electrical resistance value obtained when executing the Control 1 and a change in an electrical resistance value obtained when executing the Control 4 with each other to distinguish a response due to an alkene in a sample gas and a response due to an alcohol in a sample gas from each other and detect an alkene in a sample gas.

The control means 810 is capable of determining, in the case where a change in an electrical resistance value obtained when executing the Control 1 is significantly large and a change in an electrical resistance value obtained when executing the Control 4 is substantially zero (corresponding to the error level of the detection means 710), that an alkene is present in a sample gas, and determines, in the case where any change in an electrical resistance value is significantly large, that it is a false response due to an alcohol present in a sample gas.

It goes without saying that in the case of using the sensor 500, the control means 810 may further execute the above-mentioned Control 1 and Control 2, Control 1 and Control 3, or Control 1 to Control 3 to distinguish a response due to an alkene in a sample gas, a response due to a temperature/humidity change and/or an acid vapor, and a response due to an aldehyde and/or a ketone from each other.

The operation principle in the case where the system 800 according to the present invention includes the sensor 600 will be described.

The control means 810 controls the sensor 600 (Fig. 6) and the detection means 710 as follows.

(Control 5) The control means 810 acquires, from the detection means 710, a change in an electrical resistance value from the response unit 130-1 (Fig. 6) when a sample gas was introduced into the first flow path 610 (Fig. 6).

(Control 6) The control means 810 acquires, from the detection means 710, a change in an electrical resistance value from the response unit 130-2 (Fig. 6) when a sample gas was introduced into the second flow path 620 (Fig. 6).

(Control 7) The control means 810 acquires, from the detection means 710, a change in an electrical resistance value from the response unit 130-3 (Fig. 6) when a sample gas was introduced into the third flow path 630 (Fig. 6).

(Control 8) The control means 810 acquires, from the detection means 710, a change in an electrical resistance value from the response unit 130-4 (Fig. 6) when a sample gas was introduced into the fourth flow path (Fig. 6).

The control means 810 compares the changes in the electrical resistance value with each other to distinguish a response due to an alkene in a sample gas, a response due to a temperature/humidity change of a sample gas and/or an acid vapor, a response due to an aldehyde and/or a ketone in a sample gas, and a response due to an alcohol in a sample gas from each other and detect an alkene in a sample gas.

The control means 810 is capable of determining, in the case where a change in an electrical resistance value obtained when executing the Control 5 is significantly large and a change in an electrical resistance value obtained when executing the Controls 6 to 8 is substantially zero (corresponding to the error level of the detection means 710), that an alkene is present in a sample gas.

The control means 810 is capable of determining, in the case where a change in an electrical resistance value obtained when executing the Control 5 and the Control 6 is significantly large and a change in an electrical resistance value obtained when executing the Control 7 and the Control 8 is substantially zero (corresponding to the error level of the detection means 710), that it is a false response due to an alcohol present in a sample gas.

The control means 810 is capable of determining, in the case where a change in an electrical resistance value obtained when executing the Control 5, the Control 6, and the Control 7 is significantly large and a change in an electrical resistance value obtained when executing the Control 8 is substantially zero (corresponding to the error level of the detection means 710), that it is a false response due to an aldehyde and/or a ketone present in a sample gas.

The control means 810 is capable of determining, in the case where a change in an electrical resistance value obtained when executing the Control 5 to the Control 8 is significantly large, that it is a false response due to an acid vapor present in a sample gas and/or a temperature/humidity change of a sample gas.

Although the present invention will be described in more detail by way of Examples shown below, these are disclosed only as an aid for easily understanding the present invention and the present invention is not limited to these Examples.

(Example)

[Reagent and material]

Reagents and materials used in the following examples will be described. Unless otherwise specified, the reagents were used as they were without purification. Two types of hydroxylamine salts shown in the following formulae were used and purchased from Tokyo Chemical Industry Co., Ltd.

(Chem. 1)

$NH_2OH \cdot HCl$

Hydroxylamine hydrochloride

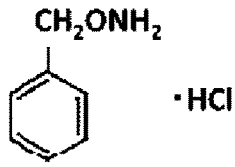

*O*-benzylhydroxylamine hydrochloride

As the semiconductor material used for the response unit 130, one of the semiconductor-rich single-wall carbon nanotube(SWCNT) described in Patent Literature 2, Purified SWCNT (PT200) from NanoC, USA, and poly(3-hexylthiophene-2,5-diyl) was used. In the case of any of the semiconductors, as an electrode, a comb-shaped electrode (manufactured by BVT Technologies, No.CC1.W1) formed of Au formed on a ceramic substrate formed of aluminum oxide was used. The distance between electrodes was 200 μm.

The semiconductor-rich SWCNT, which was prepared by a HiPco (high pressure CO) using the disproportional reaction of carbon monoxide was purchased from NanoIntegris, USA. The SWCNT was separated into a semiconductor type and a metal type on the basis of Yomogida, Y. et al.,Nat.Commun.,2016,7,12056. Further, the semiconductor-rich SWCNT was dispersed using a supramolecular polymer (n represents linear $C_8H_{17}$ and the average value of m is approximately 15 to 20) prepared on the basis of Ishihara, S. et al.,J.Am.Chem.Soc.,2016,138,8221-8227 shown in the following formula to improve the surface area.

(Chem. 2)

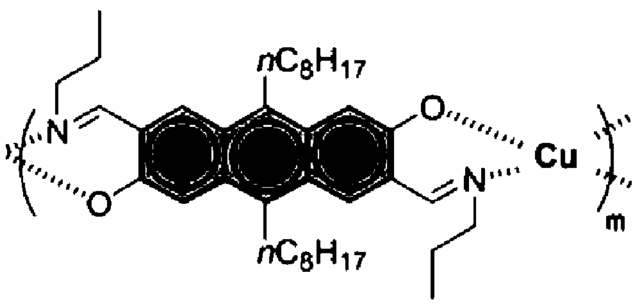

A method of synthesizing poly(3-hexylthio-phene-2,5-diyl) will be described. Poly(3-hexylthio-phene-2,5-diyl) was synthesized using cis-(2,2'-bithio-phene)-5-ylbromo[1,3-bis(diphenylphosphino)propane]nickel (II) synthesized on

the basis of E.E.Nesterov et al.,Macromolecules,2014,Vol.47,p.506-516 and 2-bromo-3-hexyl-5-iodothiophene synthesized on the basis of A.Staubitz et al.,Organic Letters,2013,Vol.15,p.4666-4669.

Description will be made in detail. A Schlenk tube containing a magnet stirrer was heated and dried under vacuum, and the atmosphere was adjusted to an argon atmosphere using a vacuum line. 99 mg of 2-bromo-3-hexyl-5-iodothiophene and 2.5 mL of dehydrated tetrahydrofuran treated with a solvent purification device (manufactured by Nikko Hansen & Co., Ltd.) were added thereto and cooled to -20°C. 214 μL of 1.3 mol/L of a tetrahydrofuran solution of an isopropylmagnesiumchlo-ride-lithium chloride complex (manufactured by Aldrich) was added dropwise to this solution at -20°C, stirred for 20 minutes, and then stirred for 70 minutes at room temperature.

0.2 mL of a dehydrated tetrahydrofuran solution of 5.4 mg of cis-(2,2'-bithiophene)-5-ylbromo[1,3-bis(diphenylphosphino)propane]nickel(II) was added to the reaction mixture, and reacted for 22 hours at room temperature. After the completion of the reaction, 1 mL of 3 mol/L of hydrochloric acid was added to the reaction mixture, stirred for 30 minutes, and extracted with chloroform. The obtained organic layer was dried with sodium sulfate, the desiccant was removed by filtration, and the solvent was distilled off under reduced pressure.

The obtained crude product was dissolved in 2.5 mL of tetrahydrofuran, added dropwise to 20 mL of methanol, and the resulting dispersoid was settled in a centrifuge. After repeating decantation of the supernatant and cleaning of the precipitate with methanol three times, the mixture was dried under reduced pressure to obtain 35 mg of a deep red solid.

The molecular weight of this solid was measured by gel permeation chromatography (GPC) measurement. Regarding the molecular weight, Mw=25100 g/mol, Mn=21200 g/mol, and Mw/Mn=1.17, and it was confirmed that poly(3-hexylthiophene-2,5-diyl) that was a polymer was synthesized. Further, the tetrahydrofuran solution of the obtained solid had an orange to red color, and had an absorption wavelength characteristic of polythiophene.

As the palladium catalyst contained in the first reaction unit, four types of Pd-$V_2O_5$-$TiO_2$ having different amounts of supported palladium were synthesized and used. Further, for comparison, also $V_2O_5$-$TiO_2$ supporting no palladium was synthesized.

A method of synthesizing the palladium catalyst will be described in detail. A magnet stirrer was placed in a 300 mL eggplant flask, and 1.0 g of a titanium dioxide ($TiO_2$) powder (manufactured by Evonik Industries, P25) and 100 mL of pure water were added thereto. The mixture was stirred for 15 minutes at room temperature. Next, 53.8 mg of ammonium metavanadate ($NH_4VO_3$) (manufactured by Sigma-Aldrich) dissolved in 10 mL of pure water was added thereto, and 0.1 M of an aqueous nitric acid solution was added thereto to adjust the pH to 4.0. The mixture was stirred for 1 hour at room temperature, and the solvent was removed by an evaporator. The residue was vacuum dried (120°C) overnight. The residue was transferred to a crucible, and heated at 400°C for 4 hours using an electric furnace. By repeating this series of processes, 2.5 g of $V_2O_5$-$TiO_2$ was obtained.

500 mg of $V_2O_5$-$TiO_2$ was placed in a crucible, and 5 sets of this were prepared. 1 mL of 0.5 M of an aqueous hydrochloric acid solution containing 0 mg, 0.84 mg, 1.68 mg, 3.37 mg, or 6.67 mg of palladium chloride was added to 5 sets of crucibles, respectively, and ultrasonic treatment was performed thereon for 10 minutes. The crucibles were treated at 400°C for 4 hours in an electric furnace. The heating rate of the electric furnace was 5°C/min. As a result, approximately 500 mg of Pd-$V_2O_5$-$TiO_2$ was obtained. The obtained five types of Pd-$V_2O_5$-$TiO_2$ were respectively named Pd-$V_2O_5$-$TiO_2$(0), Pd-$V_2O_5$-$TiO_2$ (0.84), Pd-$V_2O_5$-$TiO_2$ (1.68), Pd-$V_2O_5$-$TiO_2$ (3.37), and Pd-$V_2O_5$-$TiO_2$ (6.67), and the values in parentheses each represent the weight of palladium chloride used for synthesis. Note that Pd-$V_2O_5$-$TiO_2$(0) does not support palladium and is synonymous with $V_2O_5$-$TiO_2$.

[Example 1]

In an Example 1, an alkene-detection gas sensor 900 shown in Fig. 9A and Fig. 9B was produced and an ethylene was detected.

Fig. 9A is a schematic diagram showing an alkene-detection gas sensor (when a clean gas is introduced) according to the Example 1.

Fig. 9B is a schematic diagram showing the alkene-detection gas sensor (when a sample gas is introduced) according to the Example 1.

Pd-$V_2O_5$-$TiO_2$ (3.37) was used as the palladium catalyst 140 included in the first reaction unit 110, $NH_2OH{\cdot}HCl$ (hydroxylamine hydrochloride) was used as the hydroxylamine salts 150 included in the second reaction unit 120, and a single-wall carbon nanotube (SWCNT) containing 95 weight% of a semiconductor type and 5 weight% of a metal type, which was dispersed with the above-mentioned supramolecular polymer, was used as the semiconductor material 180 included in the response unit 130.

The first reaction unit 110 was constructed as follows. A glass tube (having an inner diameter of 5 mm and a length of 5 cm) was filled with 200 mg of a powder of Pd-$V_2O_5$-$TiO_2$ (3.37). Both ends of the glass tube were packed with glass wool so that the powder of Pd-$V_2O_5$-$TiO_2$(3.37) was held in the glass tube. The configurations of the second reaction unit 120 and the response unit 130 were the same as those of the sensor described in the Example 8 of Patent Literature 2, and the second reaction unit 120 and the response unit 130 were housed in a sensor room 920.

Gas tubes were connected to both ends of the glass tube so that a gas flows. The glass tube filled with Pd-$V_2O_5$-$TiO_2$ (3.37) was placed in an electric oven 910 (manufactured by ETTAS, IW-300S) and heated to 40°C, thereby achieving a constant temperature state. The two gas tubes connected to the both ends of the above-mentioned glass tube were taken out from the window of the electric oven 910, and a sample gas was introduced from one gas tube into the above-mentioned glass tube. The sample gas after reacting with the first reaction unit 110 was supplied to the above-mentioned second reaction unit 120 and response unit 130 through the tube on the outlet side.

The switching between a clean gas and a sample gas was performed on the rear side of the first reaction unit 110 as shown in Fig. 9A and Fig. 9B.

A method of preparing a sample gas will be described. In a 5L Tedlar (registered trademark) bag, the atmosphere that has 50% of relative humidity and contains ethylene (0.1 to 1000 ppm) was prepared. First, sample gases of 100 ppm and 1000 ppm were prepared by collecting 0.5 mL or 5 mL of pure ethylene from a push can (manufactured by GL Science) and adding it to the Tedlar bag containing 5L of the atmosphere. The concentration of ethylene was checked with a detector tube (manufactured by GASTEC CORPORATION, No.172).

Subsequently, 50 mL of 100 ppm of the sample gas was taken with a syringe, and added to the 5L Tedlar bag to prepare 1 ppm of the sample gas. 50 mL of 1000 ppm of the sample gas was taken with a syringe and added to the 5L Tedlar bag to prepare 10 ppm of the sample gas.

In the Examples of the specification of the subject patent application, a value obtained by normalizing a change in a current value by $\{(I_{(t)}-I_0)/I_0\}\times 100(\%)$ was used as a response of the sensor. Here, unless otherwise specified, $I_0$ represents a current value in the response unit 130, which is detected when the clean atmosphere (clean gas) containing no an alkene was introduced into the second reaction unit 120 and the response unit 130 bypassing the first reaction unit 110 and stabilized for 1 minute or more. $I_{(t)}$ represents a current value t seconds (t represents 300 seconds described below) after switching the gas to be introduced into the second reaction unit 120 and the response unit 130 to the sample gas that was in contact with the first reaction unit 110. Unless otherwise specified, the voltage applied to the response unit 130 was 0.1 V.

The response properties to ethylene was checked using the sensor 900 in Fig. 9. The sensor 900 was exposed to the clean atmosphere (flow rate of 25 mL/min) having 50% of humidity for 1000 seconds to stabilize the response unit 130. During this time, a sample gas (ethylene concentration: 0 ppm, 1 ppm, and 10 ppm) was introduced into the first reaction unit 110 of the sensor 900 at the flow rate of 25 mL/min using a metering pump, the clean gas and the sample gas were switched at the time point of 1000 seconds, the second reaction unit 120 and the response unit 130 were exposed to the sample gas for 300 seconds, and the change in a current value was measured. Subsequently, the sample gas was switched to the clean atmosphere again, and the clean atmosphere was exposed to the second reaction unit 120 and the response unit 130 for 700 seconds to recover the response. This process was repeated three times. The results are shown in Fig. 10.

Fig. 10 is a diagram showing response properties of a sensor according to the Example 1 to ethylene.

As shown in Part (A) of Fig. 10 to Part (C) of Fig. 10, the clean atmosphere (Part B in the figure) was caused to flow from the start of measurement to 1000 seconds to stabilize the current value of the sensor 900. After that, when a sample gas (Part A in the figure) was caused to flow for 300 seconds, a clear change (here, an increase) in a current value was observed. Subsequently, when the sample gas was switched to the clean atmosphere and the clean atmosphere (Part B in the figure) was caused to flow, the recovery of the response of the sensor was observed. This was repeated three times, and it was found that the response was reproducible.

Further, in accordance with Part (A) of Fig. 10 to Part (C) of Fig. 10, it was found that the higher the concentration of ethylene contained in the sample gas, the greater the increase in the current value in the response unit 130. This is because ethylene was oxidized to acetaldehyde by the first reaction unit 110, acetaldehyde generated an acid in the second reaction unit 120, and the acid was detected in the response unit 130.

Meanwhile, in accordance with Part (D) of Fig. 10, no clear change in a current value was observed in the case where the sample gas contains no ethylene. The observed slight change is noise or a subtle change in humidity associated with the switching operation.

Note that in the case where the atmosphere that contains 10 ppm of ethylene and has 50% of humidity was caused to pass through the above-mentioned glass tube containing 200 mg of Pd-$V_2O_5$-$TiO_2$ (3.37) placed at 40°C at the flow rate of 25 mL/min, it was confirmed by a detector tube (manufactured by GASTEC CORPORATION, 92M or 92L) that approximately 85 to 95% of ethylene was converted to acetaldehyde and the conversion efficiency was stable for at least 1 hour. Further, even in the case where Pd-$V_2O_5$-$TiO_2$ (0.84), Pd-$V_2O_5$-$TiO_2$ (1.68), or Pd-$V_2O_5$-$TiO_2$ (6.67) was used under the same condition, acetaldehyde was detected, but the conversion efficiency of ethylene was slightly (approximately 10 to 30%) lower than that of Pd-$V_2O_5$-$TiO_2$ (3.37).

From this, it was found that Pd-$V_2O_5$-$TiO_2$ (3.37) was the most suitable as a palladium catalyst used for the first reaction unit 110. Further, it was found that palladium was essential because no acetaldehyde was detected in the case of Pd-$V_2O_5$-$TiO_2$ (0).

From the above, it was shown that the alkene-detection gas sensor according to the present invention was

capable of detecting ethylene and performing repeated measurement and quantification.

[Example 2]

In an Example 2, 1-hexene that is another an alkene was detected using the aldehyde-detection gas sensor (Fig. 9) according to the Example 1. A change in a current value was measured in the same way as that in the Example 1 using a sample gas containing 860 ppm of 1-hexene. The results are shown in Fig. 11.

Fig. 11 is a diagram showing response properties of a sensor according to the Example 2 to 1-hexene.

As shown in Fig. 11, the clean atmosphere (Part B in the figure) was caused to flow from the start of measurement to 1000 seconds to stabilize the current value of the response unit 130. After that, when a sample gas (Part A in the figure) was caused to flow for 300 seconds, a clear change in a current value was observed. This is because 1-hexene was oxidized to 1-hexanal and/or 2-hexanone by the first reaction unit 110, these acetaldehyde/ketone generated an acid in the second reaction unit 120, and the acid was detected in the response unit 130. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

Although not shown, no clear change in a current value was shown for the sample gas containing no 1-hexene, similarly to Part (D) of Fig. 10.

From the above, it was shown that the alkene-detection gas sensor according to the present invention was capable of detecting various alkenes.

[Example 3]

In an Example 3, the switching device 410 (Fig. 4) was attached to the aldehyde-detection gas sensor produced in the Example 1 to produce the aldehyde-detection gas sensor shown in Fig. 4, and ethylene was detected. A change in a current value was measured in the same way as that in the Example 1 except that an introduction destination of a sample gas containing 10 ppm of ethylene was switched. The results are shown in Fig. 12.

Fig. 12 is a diagram showing response properties of a sensor according to the Example 3 to ethylene.

As shown in Fig. 12, the clean atmosphere (Part B in the figure) was caused to flow from the start of measurement to 1000 seconds to stabilize the current value of the response unit 130. During this time, when the switching device 410 was switched so that a sample gas was introduced into the second reaction unit 120 and the response unit 130 bypassing the first reaction unit 110 and the clean atmosphere and the sample gas were switched at the time point of 1000 seconds to cause the sample gas (Part A in the figure) to flow to the second reaction unit 120 for 300 seconds, a decrease in a current value was observed. This is due to the influence of a subtle change in humidity associated with the switching operation, or the like, and can be treated as a background signal because the increase/decrease in a current value differs from that of the response to ethylene in the Example 1. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

From the above, it was shown that in the alkene-detection gas sensor according to the present invention, no alkene was detected in the case of bypassing a first reaction unit.

[Example 4]

In an Example 4, the third reaction unit 510 (Fig. 5) that reacted with an alcohol to generate an aldehyde and/or a ketone was attached to the aldehyde-detection gas sensor produced in the Example 3 to produce the aldehyde-detection gas sensor shown in Fig. 5, and ethylene was detected.

A glass tube was filled with 200 mg of a $V_2O_5$-$TiO_2$ powder and both ends thereof were packed with glass wool to obtain the third reaction unit 510. A change in a current value was measured in the same way as that in the Example 1 except that an introduction destination of a sample gas containing 10 ppm of ethylene was switched. The results are shown in Fig. 13.

Fig. 13 is a diagram showing response properties of a sensor according to the Example 4 to ethylene.

As shown in Fig. 13, the clean atmosphere (Part B in the figure) was caused to flow from the start of measurement to 1000 seconds to stabilize the current value of the response unit 130. During this time, when the switching device 410 was switched so that a sample gas was introduced into the third reaction unit 510, the second reaction unit 120, and the response unit 130 bypassing the first reaction unit 110 and the gas to be introduced into the second reaction unit 120 and the response unit 130 was switched from the clean gas to the sample gas (Part A in the figure) at the time point of 1000 seconds and held for 300 seconds, a decrease in a current value was observed. This is due to the influence of

a subtle change in humidity associated with the switching operation, or the like, and the increase/decrease in a current value was opposite to that of the response to ethylene in the Example 1. From this, it is conceivable that $V_2O_5$-$TiO_2$ has no ability to convert ethylene into acetaldehyde. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

Comparison of the Example 1 and the Example 4 showed that in the alkene-detection gas sensor according to the present invention, the first reaction unit 110 needed to contain at least palladium.

[Example 5]

In Fig. 5, ethanol was detected using the alkene-detection gas sensor (Fig. 5) produced in the Example 4.

A method of preparing a sample gas will be described. In a 5L Tedlar bag, the atmosphere that has 50% of humidity and contains 500 ppm of ethanol was prepared. A saturated vapor of ethanol was collected with a syringe and added to the Tedlar bag containing 5L of the atmosphere (humidity of 50%) to prepare a sample gas of 500 ppm of ethanol. The concentration of ethanol was checked with a detector tube(manufactured by GASTEC CORPORATION, No.112L).

The response properties to ethanol were investigated using the sensor in Fig. 5. The sensor was exposed to the clean atmosphere (flow rate of 25 mL/min) having 50% of humidity for 1000 seconds to stabilize the response unit 130. Meanwhile, a sample gas containing ethanol was introduced into the first reaction unit 110 of the sensor at the flow rate of 25 mL/min using a metering pump, the clean atmosphere and the sample gas were switched at the time point of 1000 seconds and held for 300 seconds, and a change in a current value was measured. Subsequently, the clean atmosphere was exposed to the second reaction unit 120 and the response unit 130 for 700 seconds to recover the response. This process was repeated three times. The results are shown by a solid line in Fig. 14.

Next, the switching device 410 was switched so that a sample gas was introduced into the third reaction unit 510, the second reaction unit 120, and the response unit 130 bypassing the first reaction unit 110, and the response properties to ethanol were investigated in a similar procedure. The results are shown by a broken line in Fig. 14.

Next, the switching device 410 was switched so that a sample gas was introduced into the second reaction unit 120 and the response unit 130 bypassing the first reaction unit 110 and the third reaction unit 510, and the response properties to ethanol were investigated in a similar procedure. The results are shown by a dot-dash line in Fig. 14.

Fig. 14 is a diagram showing response properties of a sensor according to the Example 5 to ethanol.

The data indicated by a broken line in Fig. 14 will be described. The clean atmosphere (Part B in the figure) was caused to flow from the start of measurement to 1000 seconds to stabilize the current value of the response unit 130. After that, when a sample gas (Part A in the figure) was caused to flow for 300 seconds, a clear increase in a current value was observed. This is because ethanol was oxidized to acetaldehyde by the third reaction unit 510, acetaldehyde then generated an acid in the second reaction unit 120, and the acid was detected in the response unit 130. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

The data indicated by a solid line in Fig. 14 will be described. The clean atmosphere (Part B in the figure) was caused to flow from the start of measurement to 1000 seconds to stabilize the current value of the response unit 130. After that, when a sample gas (Part A in the figure) was caused to flow for 300 seconds, a change in a current value was observed although the change was smaller than that of the data indicated by a broken line. This is because Pd-$V_2O_5$-$TiO_2$ (3.37) has some oxidizing property to ethanol, ethanol was oxidized to acetaldehyde in the first reaction unit 110, acetaldehyde then generated an acid in the second reaction unit 120, and the acid was detected in the response unit 130. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

The data indicated by a dot-dash line in Fig. 14 will be described. The clean atmosphere (Part B in the figure) was caused to flow from the start of measurement to 1000 seconds to stabilize the current value of the response unit 130. After that, when a sample gas (Part A in the figure) was caused to flow for 300 seconds, a slight decrease in a current value was observed. This is due to the influence of the switching operation, a change in humidity, physical adsorption of organic molecules, and the like. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

From the above, combining the results of the Example 1, the Example 4, and the Example 5, it was found that the first reaction unit 110 responded to an alkene but also slightly responded to an alcohol. Meanwhile, it was found that the third reaction unit 510 responded to an alcohol but did not respond to an alkene.

From these, it was shown that by using the third reaction unit 510 that reacts with an alcohol to generate an

aldehyde and/or a ketone, it was possible to distinguish a response due to an alkene in a sample gas and a response due to an alcohol in a sample gas from each other and detect an alkene in a sample.

[Example 6]

In Fig. 6, acetaldehyde was detected using the alkene-detection gas sensor (Fig. 4) produced in the Example 3.

A method of preparing a sample gas will be described. In a 5L Tedlar bag, the atmosphere that has 50% of humidity and contains 10 ppm of acetaldehyde was prepared. First, a saturated vapor of acetaldehyde was collected with a syringe and added to the Tedlar bag containing 5L of the atmosphere (humidity of 50%) to prepare a sample gas of 1000 ppm of acetaldehyde. The concentration of acetaldehyde was checked with a detector tube (manufactured by GASTEC CORPORATION, No.92M). Subsequently, 50 mL of the atmosphere of 1000 ppm of acetaldehyde was collected with a syringe and added to the Tedlar bag containing 5L of the atmosphere (humidity of 50%) to prepare a sample gas of 10 ppm of acetaldehyde. The concentration of acetaldehyde was checked with a detector tube (manufactured by GASTEC CORPORATION, No.92L).

The response properties to acetaldehyde were investigated using the sensor in Fig. 4. The sensor was exposed to the clean atmosphere (flow rate of 25 mL/min) having 50% of humidity for 1000 seconds to stabilize the response unit 130. Meanwhile, a sample gas containing acetaldehyde was introduced into the first reaction unit 110 of the sensor at the flow rate 25mL/min using a metering pump, the clean atmosphere and the sample gas were switched at the time point of 1000 seconds and held for 300 seconds, and a change in a current value was measured. Subsequently, the clean atmosphere was exposed to the second reaction unit 120 and the response unit 130 for 700 seconds to recover the response. This process was repeated three times. The results are shown by a solid line in Fig. 15.

Next, the switching device 410 was switched so that a sample gas was introduced into the second reaction unit 120 and the response unit 130 bypassing the first reaction unit 110, and the response properties to acetaldehyde were investigated in a similar procedure. The results are shown by a broken line in Fig. 15.

Fig. 15 is a diagram showing response properties of a sensor according to the Example 6 to acetaldehyde.

The data indicated by a solid line in Fig. 15 will be described. The clean atmosphere (Part B in the figure) was caused to flow from the start of measurement to 1000 seconds to stabilize the current value of the response unit 130. After that, when a sample gas (Part A in the figure) was caused to flow for 300 seconds, an increase in a current value was observed. This is because acetaldehyde passed through the first reaction unit 110, acetaldehyde then generated an acid in the second reaction unit 120, and the acid was detected in the response unit 130. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

The data indicated by a broken line in Fig. 15 will be described. The clean atmosphere (Part B in the figure) was caused to flow from the start of measurement to 1000 seconds to stabilize the current value of the response unit 130. After that, when a sample gas (Part A in the figure) was caused to flow for 300 seconds, an increase in a current value was observed. This is because acetaldehyde generated an acid in the second reaction unit 120 and the acid was detected in the response unit 130. Subsequently, when the sample gas was switched to the clean atmosphere (Part B in the figure) and the clean atmosphere was caused to flow for 700 seconds, the recovery of the response of the sensor was observed. This was repeated three times, and it was found that the response was reproducible.

Further, in accordance with Fig. 15, it is conceivable that a response (solid line) in the case of passing through the first reaction unit 110 and a response (broken line) in the case of bypassing the first reaction unit 110 are substantially the same, and acetaldehyde is capable of quantitatively passing through the first reaction unit 110 without being adsorbed thereon.

From the above, combining the results of the Example 1, the Example 3, and the Example 6, it was found that the first reaction unit 110 reacted to an alkene but showed a similar response to a carbonyl compound such as an aldehyde/a ketone because it caused the carbonyl compound to pass through the second reaction unit 120. Meanwhile, it was found that the second reaction unit 120 responded to a carbonyl compound but did not respond to an alkene.

From these, by comparing a response in the case of using the first reaction unit 110, the second reaction unit 120, and the response unit 130 and a response in the case of using the second reaction unit 120 and the response unit 130 bypassing the first reaction unit 110 with each other, it is possible to distinguish a response due to an alkene in a sample gas and a response due to a carbonyl compound in a sample gas from each other and detect an alkene in a sample.

[Example 7]

In an Example 7, responses to toluene, acetonitrile, tetrahydrofuran, deuterated chloroform, and methane were investigated using the aldehyde-detection gas sensor (Fig. 9) according to the Example 1. Toluene, acetonitrile, tetrahydrofuran, deuterated chloroform, and methane used here are examples of a compound having a functional group that

is chemically expected not to generate an acid even in the case where it acts on the first reaction unit 110 and the second reaction unit 120. Note that since commercially available chloroform contains ethanol as a stabilizer, deuterated chloroform for measuring NMR, which does not contain ethanol, was used in this example.

In a 5L Tedlar bag, the atmosphere that contains 120 ppm of toluene, 600 ppm of acetonitrile, 800 ppm of tetrahydrofuran, 1020 ppm of chloroform, or 1000 ppm of methane and has relative humidity of 50% was prepared. The sample gas containing toluene, acetonitrile, tetrahydrofuran, or chloroform was generated by a diffusion tube method, and the concentration was calculated from the decrease amount of weight of a solvent. The sample gas containing methane was prepared in the same way as that of ethylene described in the Example 1. A change in a current value was measured in the same way as that in the Example 1 using the sample gas prepared in this way. The results are shown in Fig. 16.

Fig. 16 is a diagram showing response properties of a sensor according to the Example 7 to toluene, acetonitrile, tetrahydrofuran, deuterated chloroform, and methane.

As shown in Part (A) of Fig. 16 to Part (E) of Fig. 16, the clean atmosphere (Part B in the figure) was caused to flow from the start of measurement to 1000 seconds to stabilize the current value of the sensor 900. After that, when each sample gas (Part A in the figure) that had been caused to pass through the first reaction unit 110 was caused to flow for 300 seconds, a decrease in a current value was observed. This is a response associated with the switching operation or molecules physically adsorbed on a semiconductor material, and is opposite to a response observed in the case of an alkene. Subsequently, when the sample gas was switched to the clean atmosphere and the clean atmosphere (Part B in the figure) was caused to flow for 700 seconds, the recovery of the response of the sensor was observed. This was repeated three times, and it was found that the response was reproducible.

From the above, it was shown that the sensor according to the present invention did not respond to a compound (e.g., toluene, acetonitrile, tetrahydrofuran, deuterated chloroform, and methane) that did not generate an acid even in the case where it acted on the first reaction unit 110 and the second reaction unit 120 and an alkene could be accurately detected.

[Example 8]

In an Example 8, an alkene contained in an inert gas that contains no oxygen was detected using the aldehyde-detection gas sensor (Fig. 9) according to the Example 1.

In a 5L Tedlar bag, nitrogen that has 50% of relative humidity and contains ethylene (10 ppm) was prepared. First, 5 mL of pure ethylene was collected with a syringe from a push can (manufactured by GL Science) and added to the Tedlar bag containing 5L of nitrogen to prepare 1000 ppm of a sample gas. Subsequently, 1000 ppm of a sample gas was collected with a 50 mL syringe and added to a 5L Tedlar bag to prepare 10 ppm of a sample gas.

A change in a current value was measured in the same way as that in the Example 1 using the sample gas prepared in this way except that clean nitrogen (humidity of 50%) was used instead of the clean atmosphere. The results are shown in Fig. 17.

Fig. 17 is a diagram showing response properties of a sensor according to the Example 8 to ethylene.

The clean nitrogen (Part B in the figure) was caused to flow from the start of measurement to 1000 seconds to stabilize the current value of the sensor 900. After that, when a sample gas (Part A in the figure) was caused to flow for 300 seconds, a clear increase in a current value was observed. This is because ethylene in nitrogen was oxidized to acetaldehyde by the first reaction unit 110, the acetaldehyde generated an acid in the second reaction unit 120, and the acid was detected in the response unit 130. Subsequently, when the sample gas was switched to the clean nitrogen and the clean nitrogen (Part B in the figure) was caused to flow for 700 seconds, the recovery of the response of the sensor was observed. This was repeated three times, and it was found that the response was reproducible. Under this measurement condition, it is understood that $V_2O_5$ contained in Pd-$V_2O_5$-$TiO_2$ in the first reaction unit 110 functions as an oxidizing agent.

From the above, it was shown that the alkene-detection gas sensor according to the present invention was capable of detecting an alkene in an inert atmosphere containing no oxygen.

[Example 9]

In an Example 9, ethylene was detected using the aldehyde-detection gas sensor (Fig. 4) produced in the Example 3 and only a sample gas containing ethylene without using a clean atmosphere containing no ethylene.

A change in a current value was measured in the same way as that in the Example 1 except that an introduction destination of a sample gas containing 10 ppm of ethylene was switched. The results are shown in Fig. 18.

Fig. 18 is a diagram showing response properties of a sensor according to the Example 9 to ethylene.

First, the switching device 410 was switched so that a sample gas was introduced into the second reaction unit 120 and the response unit 130 bypassing the first reaction unit 110, and the sample gas was exposed to the second

reaction unit 120 for 2000 seconds (Part B in the figure) to stabilize the response unit 130. Since the first reaction unit 110 is bypassed, ethylene is not converted into acetaldehyde and no response is observed.

After 2000 seconds from the start of measurement, the switching device 410 was switched so that a sample gas was introduced into the first reaction unit 110, the second reaction unit 120, and the response unit 130, and the sample gas (Part A in the figure) was caused to flow for 300 seconds. As a result, a rise (increase) in a current value was observed in the response unit 130. This is because ethylene was converted into acetaldehyde in the first reaction unit 110, acetaldehyde then generated an acid in the second reaction unit 120, and the acid was detected in the response unit 130.

Subsequently, when the switching device 410 was switched so that the sample gas was introduced into the second reaction unit 120 and the response unit 130 bypassing the first reaction unit 110 again and the sample gas (Part B in the figure) was caused to flow through the second reaction unit 120 and held to the time point of 8000 seconds, the recovery of the response of the sensor was observed.

From the above, it was shown that by using a switching device and bypassing a first reaction unit in the alkene-detection gas sensor according to the present invention, it was possible to detect an alkene using only a sample gas without using a clean atmosphere.

[Example 10]

In an Example 10, an alkene-detection gas sensor 1900 shown in Fig. 19 was produced and ethylene was detected.

Fig. 19 is a schematic diagram showing an alkene-detection gas sensor according to the Example 10.

Pd-$V_2O_5$-$TiO_2$ (6.67) was used as the palladium catalyst 140 contained in the first reaction unit 110, O-benzylhydroxylamine hydrochloride ($NH_2OCH_2Ph \cdot HCl$) was used as the hydroxylamine salts 150 contained in the second reaction unit 120, and Purified SWCNT (PT200) manufactured by NanoC, USA, was used as the semiconductor material 180 contained in the response unit 130. The first reaction unit 110 was constructed in the same way as that in the Example 1.

Fig. 20 is a diagram showing the procedure for producing a second reaction unit according to the Example 10.

As the second reaction unit 120, O-benzylhydroxylamine hydrochloride (manufactured by Tokyo Chemical Industry Co., Ltd) was used as hydroxylamine salts and a type sandwiching and encapsulating a solid powder of hydroxylamine salts in a porous filter was adopted.

A circular hole having a diameter of 4 mm in a vinyl tape (manufactured by Scotch) that has a thickness of 0.2 mm and is formed of poly-vinyl chloride using a leather punch, and a hydrophobic membrane filter (manufacture by Advantec, a hole diameter of 0.2 micrometer, T020A047A) of polytetrafluoroethylene (PTFE) hollowed out to a diameter of 6 mm was attached to the adhesive surface side to fill the hole of the vinyl tape without gaps. Two sets of a construct including this vinyl tape and a hydrophobic membrane filter were prepared, approximately 2 mg of a powder of O-benzylhydroxylamine hydrochloride was placed on the one hydrophobic membrane filter portion (on the side without the adhesive surface of the vinyl tape), and the other construct was caused to cover thereon so that O-benzylhydroxylamine hydrochloride was sandwiched between two membrane filters to adhere the two constructs to each other. As a result, the solid of O-benzylhydroxylamine hydrochloride is sandwiched and held by the porous filter, and a vapor of an acid generated by the reaction with formaldehyde is capable of passing through the porous filter to reach the response unit 130.

The response unit 130 was constructed as follows. A SWCNT (21.9 mg) was suspended in 29.1 mL of o-dichlorobenzene (o-DCB). Ultrasonic treatment was performed on the suspension at room temperature for 30 minutes. The obtained suspension (approximately 0.5 mL) was diluted 10-fold with o-DCB, and ultrasonic treatment was performed thereon at room temperature for further 30 minutes to obtain o-DCB containing 0.1 mg/mL of a SWCNT. The SWCNT was dispersed and no agglomeration of the SWCNT was visually observed. Approximately 1 microliter was drop cast onto a comb-shaped electrode, and o-DCB was removed by drying. When 6 sensors were prepared and electrical resistance values were measured by an electric resistance meter, the electrical resistance values were within the range of 30 to 80 kΩ. By rubbing the electrode portion with a cotton swab and removing part of the SWCNT, the electrical resistance values were adjusted to approximately 150 kΩ to unify the resistance values of the sensors.

The second reaction unit 120 was installed above the response unit 130 with a spacer that has a thickness of 0.2 mm and is formed of a vinyl tape material sandwiched therebetween and housed in the sensor room 920.

Gas tubes were connected to both ends of a glass tube to allow a gas to flow. The glass tube filled with Pd-$V_2O_5$-$TiO_2$ (6.67) was placed in the electric oven 910. The two gas tubes connected to the both ends of the above-mentioned glass tube were taken out from the window of the electric oven 910, and a sample gas was introduced from one gas tube into the above-mentioned glass tube. The sample gas after reacting with the first reaction unit 110 was supplied to the above-mentioned second reaction unit 120 and response unit 130 through the tube on the outlet side. Note that the experiment was conducted at room temperature (20°C) without performing heating by the electric oven 910.

In the Example 10, the switching between a clean gas and a sample gas was performed on the front stage of

the first reaction unit 110 as shown in Fig. 19. For this reason, it should be noted that it takes time for the gas to pass through the first reaction unit 110 and reach the second reaction unit 120 and the response unit 130 and there is a time lag between the switching operation of the gas and the response of the sensor. The time difference depends on the length of the flow path and the flow rate of the sample gas.

A change in a current value was measured in the same way as that in the Example 1 except that a clean atmosphere having relative humidity of 65, a sample gas that has relative humidity of 65 and contains 10 ppm of ethylene, and the voltage of 1.0 V applied to the response unit 130 were used. The results are shown in Fig. 21.

Fig. 21 is a diagram showing response properties of a sensor according to the Example 10 ethylene.

As shown in Fig. 21, the clean atmosphere (Part B in the figure) was caused to flow from the start of measurement to 1000 seconds to stabilize the current value of the response unit 130. After that, when a sample gas (Part A in the figure) was caused to flow for 500 seconds, an increase in a current value was observed. This is because ethylene was oxidized to acetaldehyde in the first reaction unit 110, acetaldehyde generated an acid in the second reaction unit 120, and the acid was detected in the response unit 130. Subsequently, when the sample gas was switched to the clean atmosphere and the clean atmosphere (Part B in the figure) was held to the time point of 3000 seconds, the recovery of the response of the sensor was observed.

From the above, it was shown that in the alkene-detection gas sensor according to the present invention, there was a degree of freedom in the amount of palladium used in the first reaction unit 110, the first reaction unit 110 could be used at room temperature, powdered hydroxylamine salts ($NH_2OR$) could be used for the second reaction unit 120, and a carbon material different from that in the Example 1 could be used in the response unit 130.

[Example 11]

In an Example 11, an alkene-detection gas sensor was produced in the same way as that in the Example 10 except that Pd-$V_2O_5$-$TiO_2$ (0.84) was used as the palladium catalyst 140 contained in the first reaction unit 110, hydroxylamine hydrochloride ($NH_2OH{\cdot}HCl$) was used as the hydroxylamine salts 150 contained in the second reaction unit 120, and poly(3-hexylthiophene-2,5-diyl) was used as the semiconductor material 180 contained in the response unit 130 in the alkene-detection gas sensor shown in Fig. 19, and ethylene was detected. The first reaction unit 110 was constructed in the same way as that in the Example 1.

Hydroxylamine hydrochloride was added to methanol until saturated. A drop of the supernatant solution was drop cast onto a hydrophilic PTFE membrane filter (Omnipore manufactured by Merck Millipore, a hole diameter of 0.2 μm, JGWP04700). Methanol was dried in the atmosphere, and a rectangular piece having a width of 6 mm and a height of 3 mm was cut out to produce the second reaction unit 120. The amount of hydroxylamine hydrochloride supported on the second reaction unit 120 was estimated to be approximately 0.5 mg.

0.6 mL of tetrahydrofuran was added to 0.45 mg of poly(3-hexylthiophene-2,5-diyl), and was completely dissolved by ultrasonic treatment and heating to approximately 50°C. Approximately 0.5 microliters of the obtained solution was drop cast onto a comb-shaped electrode, and tetrahydrofuran was removed by drying. 6 sensors were prepared and left to stand in the atmosphere for 1 week. When electrical resistance values were measured by an electric resistance meter, they were approximately 4 to 6 MΩ. In this way, the response unit 130 of the sensor was produced.

The second reaction unit 120 was installed above the response unit 130 with a spacer that has a thickness of 0.2 mm and is formed of a vinyl tape material sandwiched therebetween and housed in the sensor room 920.

A change in a current value was measured in the same way as that in the Example 10 except that a clean atmosphere having relative humidity of 58% and a sample gas that has relative humidity of 58% and contains 10 ppm of ethylene were used. The results are shown in Fig. 22.

Fig. 22 is a diagram showing response properties of a sensor to ethylene according to the Example 11.

As shown in Fig. 22, the clean atmosphere (Part B in the figure) was caused to flow from the start of measurement to 1000 seconds to stabilize the current value of the response unit 130. After that, when a sample gas (Part A in the figure) was caused to flow for 500 seconds, an increase in a current value was observed. This is because ethylene was oxidized to acetaldehyde in the first reaction unit 110, acetaldehyde generated an acid in the second reaction unit 120, and the acid was detected in the response unit 130. Subsequently, when the sample gas was switched to the clean atmosphere and the clean atmosphere (Part B in the figure) was held to the time point of 4000 seconds, the recovery of the response of the sensor was observed.

When the sample gas (Part A in the figure) was caused to flow for 500 seconds again, an increase in a current value was observed. When the clean atmosphere was held to the time point of 4500 seconds, the recovery of the response of the sensor was observed and it was confirmed that the response was reproducible.

From the above, it was shown that in the alkene-detection gas sensor according to the present invention, various semiconductor materials could be used for the response unit 130.

Industrial Applicability

The alkene-detection gas sensor according to the present invention and the system using the same are capable of selectively and highly-sensitively detecting an alkene by a change in an electrical resistance value. Since the sensor and the system are compact and are capable of constantly monitoring an alkene, they function as a detection gas sensor for detecting an alkene and controlling the concentration. The compact sensor and system capable of constantly monitoring ethylene that is a plant hormone can be useful for management of force-ripening, storage, transportation, and the like of fruits and vegetables.

Reference Signs List

| | |
|---|---|
| 100, 200, 300, 400, 500, 600, 900, 1900 | al- kene-detection gas sensor |
| 110 | first reaction unit |
| 120 | second reaction unit |
| 130 | response unit |
| 140 | palladium catalyst |
| 150 | hydroxylamine salts |
| 160 | substrate |
| 170 | electrode |
| 180 | semiconductor material |
| 190 | spacer |
| 410 | switching device |
| 510 | third reaction unit |
| 610 | first flow path |
| 620 | second flow path |
| 630 | third flow path |
| 640 | fourth flow path |
| 700, 800 | alkene detection system |
| 710 | detection means |
| 720 | power source |
| 810 | control means |
| 910 | electric oven |
| 920 | sensor room |

**Claims**

**1.** An alkene-detection gas sensor that detects an alkene in a sample gas, comprising:

a first reaction unit that contains a palladium catalyst and oxidizes an alkene in a sample gas to convert the alkene into an aldehyde and/or a ketone;
a second reaction unit that contains hydroxylamine salts and reacts with the aldehyde and/or ketone converted in the first reaction unit to generate an acid; and
a response unit that includes an electrode supporting a semiconductor material of which an electrical resistance value changes by the generated acid, wherein
the palladium catalyst, the hydroxylamine salts, and the semiconductor material are separated from each other.

**2.** The sensor according to claim 1, wherein
the alkene is ethylene.

**3.** The sensor according to claim 1 or 2, wherein
the palladium catalyst is a solid catalyst in which metal palladium (Pd) or a palladium ion ($Pd^{2+}$) is supported on an inorganic solid substance.

**4.** The sensor according to claim 3, wherein
the inorganic solid substance is selected from at least one of the group consisting of $V_2O_5$-$TiO_2$, $CeO_2$-$TiO_2$,

$V_2O_5$-$CeO_2$, $V_2O$-zeolite, $CeO_2$-zeolite, $V_2O_5$-$SiO_2$, $CeO_2$-$SiO_2$, $V_2O_5$-$Al_2O_3$, $CeO_2$-$Al_2O_3$, $Cu_2O$-$TiO_2$, $Cu_2O$-$TiO_2$, CuO-$TiO_2$, $Cu_2O$-zeolite, $Cu_2O$-$SiO_2$, CuO-zeolite, CuO-$SiO_2$, $Cu_2O$-$Al_2O_3$, CuO-$Al_2O_3$, $V_2O_5$-silica alumina, $CeO_2$-silica alumina, $Cu_2O$-silica alumina, CuO-silica alumina, $V_2O_5$-ZnO, $CeO_2$-ZnO, $Cu_2O$-ZnO, CuO-ZnO, $V_2O_5$-$ZrO_2$, $CeO_2$-$ZrO_2$, $Cu_2O$-$ZrO_2$, CuO-$ZrO_2$, $V_2O_5$-$WO_3$, $CeO_2$-$WO_3$, $Cu_2O$-$WO_3$, and CuO-$WO_3$.

**5.** The sensor according to claim 4, wherein

the solid catalyst is represented by the following general formula (1).

$$(Pd+Pd^{2+})_x(V_2O_5)_y(TiO_2)_z \qquad (1)$$

x, y, and z in the formula (1) are numbers satisfying relationships of $0.0001 \leq x \leq 0.1$, $0.001 \leq y \leq 0.5$, $0.40 \leq z \leq 0.998$, and $x+y+z=1$.

**6.** The sensor according to any one of claims 3 to 5, wherein

the first reaction unit includes a column housing the solid catalyst, and
the sample gas is introduced into the column.

**7.** The sensor according to any one of claims 3 to 6, wherein

the solid catalyst is a powder, and
the solid catalyst is supported or encapsulated in a porous material selected from the group consisting of paper, cellulose, a hydrophobic polymer, a hydrophilic polymer, porous glass, glass fiber, a porous carbon material, and a porous oxide.

**8.** The sensor according to any one of claims 1 to 7, wherein
the hydroxylamine salts represent a salt obtained by neutralizing hydroxylamine ($NH_2OH$) or a hydroxylamine derivative ($NH_2OR$, where R represents an aromatic, cyclic, or acyclic hydrocarbon compound or a derivative thereof) with an acid selected from the group consisting of hydrogen halide, nitric acid, sulfuric acid, phosphoric acid, boric acid, and trifluoroacetic acid.

**9.** The sensor according to any one of claims 1 to 8, wherein
the hydroxylamine salts are encapsulated in a porous filter.

**10.** The sensor according to claim 9, wherein
the porous filter is selected from the group consisting of paper, cellulose, a hydrophobic polymer, a hydrophilic polymer, porous glass, glass fiber, a porous carbon material, and a porous oxide.

**11.** The sensor according to claim 9 or 10, further comprising
a spacer between the second reaction unit and the response unit.

**12.** The sensor according to any one of claims 7 and 9 to 11, further comprising
a space between the first reaction unit and the second reaction unit.

**13.** The sensor according to any one of claims 1 to 12, wherein
the semiconductor material is a carbon material.

**14.** The sensor according to any one of claims 1 to 13, wherein
the carbon material is selected from the group consisting of carbon nanotube, carbon nanohorn, graphene, fullerene, and derivatives thereof.

**15.** The sensor according to claim 14, wherein
the carbon nanotube is a mixture of a semiconductor type single-wall carbon nanotube and a metal type carbon nanotube, and a content ratio of the semiconductor type single-wall carbon nanotube to the metal type carbon nanotube is larger than 2.

**16.** The sensor according to any one of claims 1 to 15, further comprising

a heating device that heats the first reaction unit.

**17.** The sensor according to any one of claims 1 to 16, further comprising
a humidifying device that is located in front of the first reaction unit and humidifies the sample gas.

**18.** The sensor according to any one of claims 1 to 17, further comprising

a switching device that switches an introduction destination of the sample gas, wherein
the switching device switches between introducing the sample gas into the first reaction unit, the second reaction unit, and the response unit in this order, introducing the sample gas into the second reaction unit and the response unit in this order without introducing the sample gas into the first reaction unit, and introducing the sample gas into the response unit without introducing the sample gas into the first reaction unit and the second reaction unit.

**19.** The sensor according to any one of claims 1 to 17, further comprising:

a third reaction unit that contains at least an oxidizing agent or an oxidation catalyst and reacts with an alcohol to generate an aldehyde and/or a ketone; and
a switching device that switches an introduction destination of the sample gas, wherein
the switching device switches between introducing the sample gas into the first reaction unit, the second reaction unit, and the response unit in this order without introducing the sample gas into the third reaction unit, introducing the sample gas into the second reaction unit and the response unit in this order without introducing the sample gas into the first reaction unit and the third reaction unit, introducing the sample gas into the response unit without introducing the sample gas into the first reaction unit, the second reaction unit, and the third reaction unit, and introducing the sample gas into the third reaction unit, the second reaction unit, and the response unit in this order without introducing the sample gas into the first reaction unit.

**20.** The sensor according to any one of claims 1 to 17, further comprising:

a third reaction unit that contains at least an oxidizing agent or an oxidation catalyst and reacts with an alcohol to generate an aldehyde and/or a ketone;
a first flow path that includes the first reaction unit, the second reaction unit, the response unit connected in series, the sample gas being introduced into the first reaction unit in the first flow path;
a second flow path that includes the third reaction unit, the second reaction unit, and the response unit connected in series, the sample gas being introduced into the third reaction unit in the second flow path;
a third flow path that includes the second reaction unit and the response unit connected in series, the sample gas being introduced into the second reaction unit in the third flow path; and
a fourth flow path that includes the response unit, the sample gas being introduced into the response unit.

**21.** The sensor according to claim 19 or 20, wherein
the oxidizing agent or the oxidation catalyst contains at least $V_2O_5$.

**22.** An alkene detection system, comprising:

an alkene-detection gas sensor that detects an alkene in a sample gas; and
a detection means, wherein
the alkene-detection gas sensor is the alkene-detection gas sensor according to any one of claims 1 to 17, and the detection means detects a change in an electrical resistance value from the alkene-detection gas sensor.

**23.** The system according to claim 22, further comprising
a control means that controls operations of the alkene-detection gas sensor and the detection means.

**24.** The system according to claim 23, wherein
the alkene-detection gas sensor further includes a switching device that switches an introduction destination of the sample gas.

**25.** The system according to claim 24, wherein

the alkene-detection gas sensor further includes a third reaction unit that contains at least an oxidizing agent or an oxidation catalyst and reacts with an alcohol to generate an aldehyde and/or a ketone, and
the control means

controls an operation of the switching device to control a connection state of the first reaction unit, the second reaction unit, the third reaction unit, and the response unit and select at least two or more different connection states,
acquires, from the detection means, a change in an electrical resistance value from the response unit measured in each of the selected at least two or more different connection states, and
compares the acquired changes in the electrical resistance value with each other to detect an alkene in the sample gas.

**26.** The system according to claim 24 or 25, wherein
the control means

controls the switching device to introduce the sample gas into the first reaction unit, the second reaction unit, and the response unit in this order, and acquires, from the detection means, a change in an electrical resistance value from the response unit when the sample gas was introduced into the first reaction unit,
controls the switching device to introduce the sample gas into the response unit without introducing the sample gas into the first reaction unit and the second reaction unit, and acquires, from the detection means, a change in an electrical resistance value from the response unit when the sample gas was introduced into the response unit, and
compares the changes in the electrical resistance value with each other to distinguish a response due to an alkene in the sample gas and a response due to a temperature/humidity change and/or an acid vapor from each other and detect an alkene in the sample gas.

**27.** The system according to any one of claims 24 to 26, wherein
the control means

controls the switching device to introduce the sample gas into the first reaction unit, the second reaction unit, and the response unit in this order, and acquires, from the detection means, a change in an electrical resistance value from the response unit when the sample gas was introduced into the first reaction unit,
controls the switching device to introduce the sample gas into the second reaction unit and the response unit without introducing the sample gas into the first reaction unit, and acquires, from the detection means, a change in an electrical resistance value from the response unit when the sample gas was introduced into the second reaction unit, and
compares the changes in the electrical resistance value with each other to distinguish a response due to an alkene in the sample gas and a response due to an aldehyde and/or a ketone in the sample gas from each other and detect an alkene in the sample gas.

**28.** The system according to any one of claims 24 to 27, wherein

the alkene-detection gas sensor further includes a third reaction unit that contains at least an oxidizing agent or an oxidation catalyst and reacts with an alcohol to generate an aldehyde and/or a ketone, and
the control means

controls the switching device to introduce the sample gas into the first reaction unit, the second reaction unit, and the response unit in this order, and acquires, from the detection means, a change in an electrical resistance value from the response unit when the sample gas was introduced into the first reaction unit,
controls the switching device to introduce the sample gas into the third reaction unit, the second reaction unit, and the response unit in this order without introducing the sample gas into the first reaction unit, and acquires, from the detection means, a change in an electrical resistance value from the response unit when the sample gas was introduced into the third reaction unit, and
compares the changes in the electrical resistance value with each other to distinguish a response due to an alkene in the sample gas and a response due to an alcohol in the sample gas with each other and detect an alkene in the sample gas.

**29.** The system according to claim 23, wherein

the alkene-detection gas sensor further includes

a third reaction unit that contains at least an oxidizing agent or an oxidation catalyst and reacts with an alcohol to generate an aldehyde and/or a ketone,
a first flow path that includes the first reaction unit, the second reaction unit, the response unit connected in series, the sample gas being introduced into the first reaction unit in the first flow path,
a second flow path that includes the third reaction unit, the second reaction unit, and the response unit connected in series, the sample gas being introduced into the third reaction unit in the second flow path,
a third flow path that includes the second reaction unit and the response unit connected in series, the sample gas being introduced into the second reaction unit in the third flow path, and
a fourth flow path that includes the response unit, the sample gas being introduced into the response unit, and

the control means

acquires, from the detection means, a change in an electrical resistance value from the response unit of the first flow path when the sample gas was introduced into the first flow path,
acquires, from the detection means, a change in an electrical resistance value from the response unit of the second flow path when the sample gas was introduced into the second flow path,
acquires, from the detection means, a change in an electrical resistance value from the response unit of the third flow path when the sample gas was introduced into the third flow path,
acquires, from the detection means, a change in an electrical resistance value from the response unit of the fourth flow path when the sample gas was introduced into the fourth flow path, and
compares the changes in the electrical resistance value with each other to distinguish a response due to an alkene in the sample gas, a response due to a temperature/humidity change of the sample gas and/or an acid vapor, a response due to an aldehyde and/or a ketone in the sample gas, and a response due to an alcohol in the sample gas from each other and detect an alkene in the sample gas.

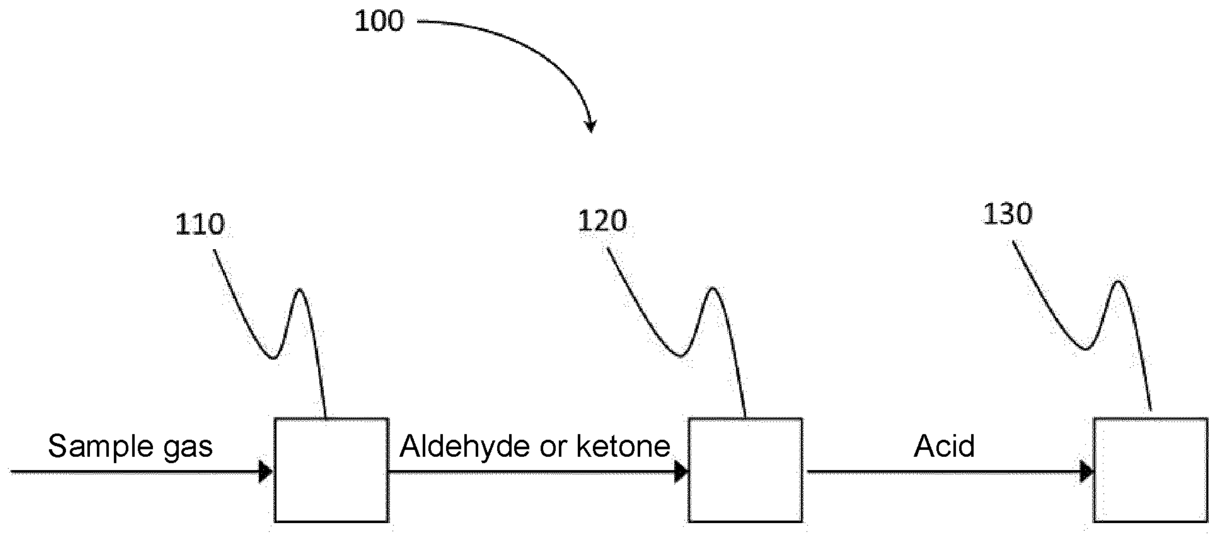
100
110
120
130
Sample gas
Aldehyde or ketone
Acid

FIG.1

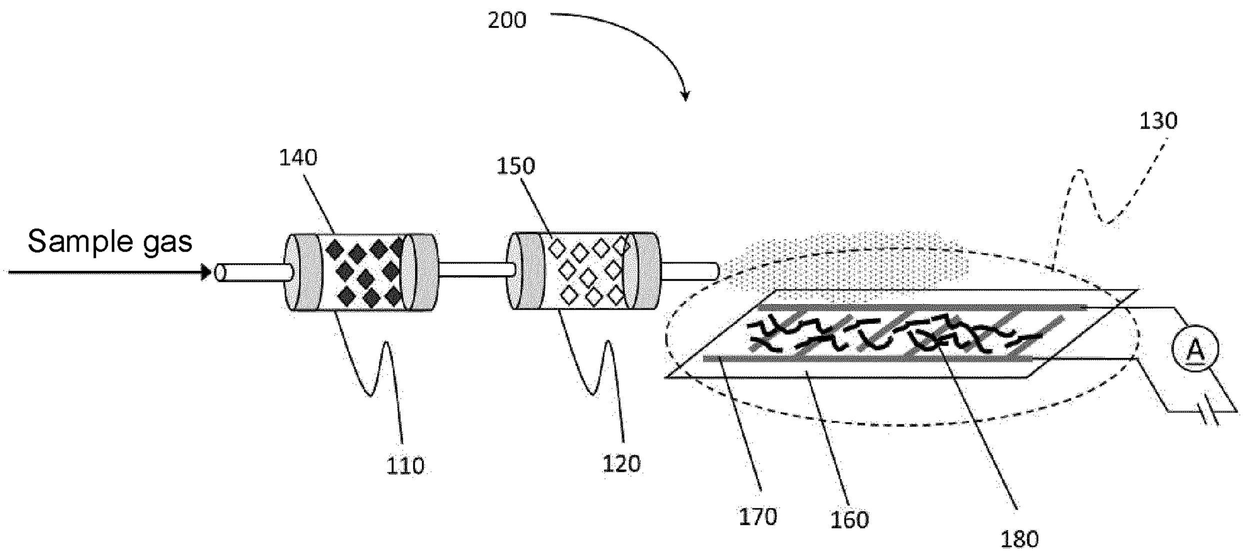
200
130
140
150
Sample gas
A
110
120
170
160
180

FIG.2

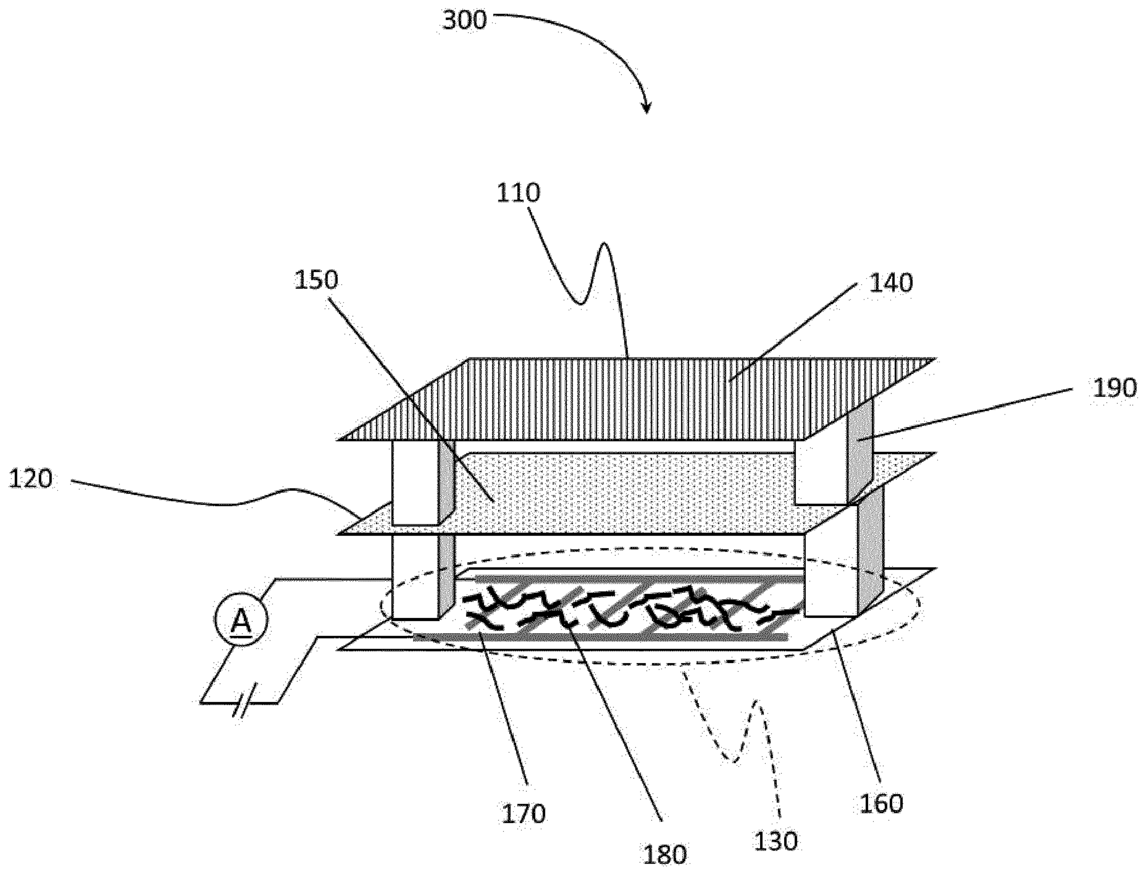
300
110
150
140
190
120
A
170
180
130
160

FIG.3

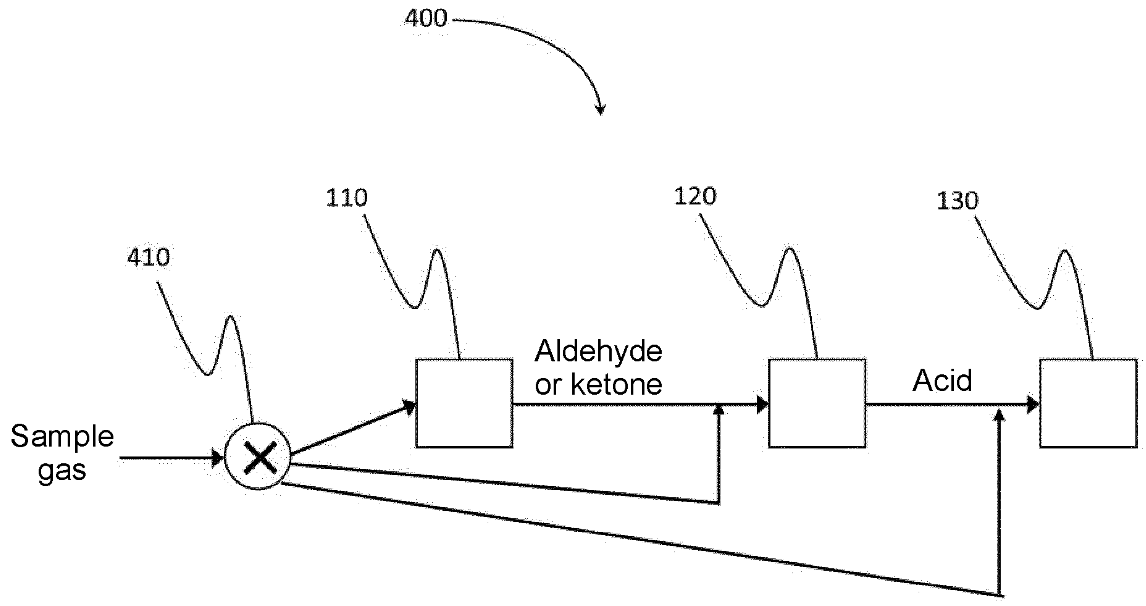
400
110
120
130
410
Aldehyde
or ketone
Acid
Sample
gas

FIG.4

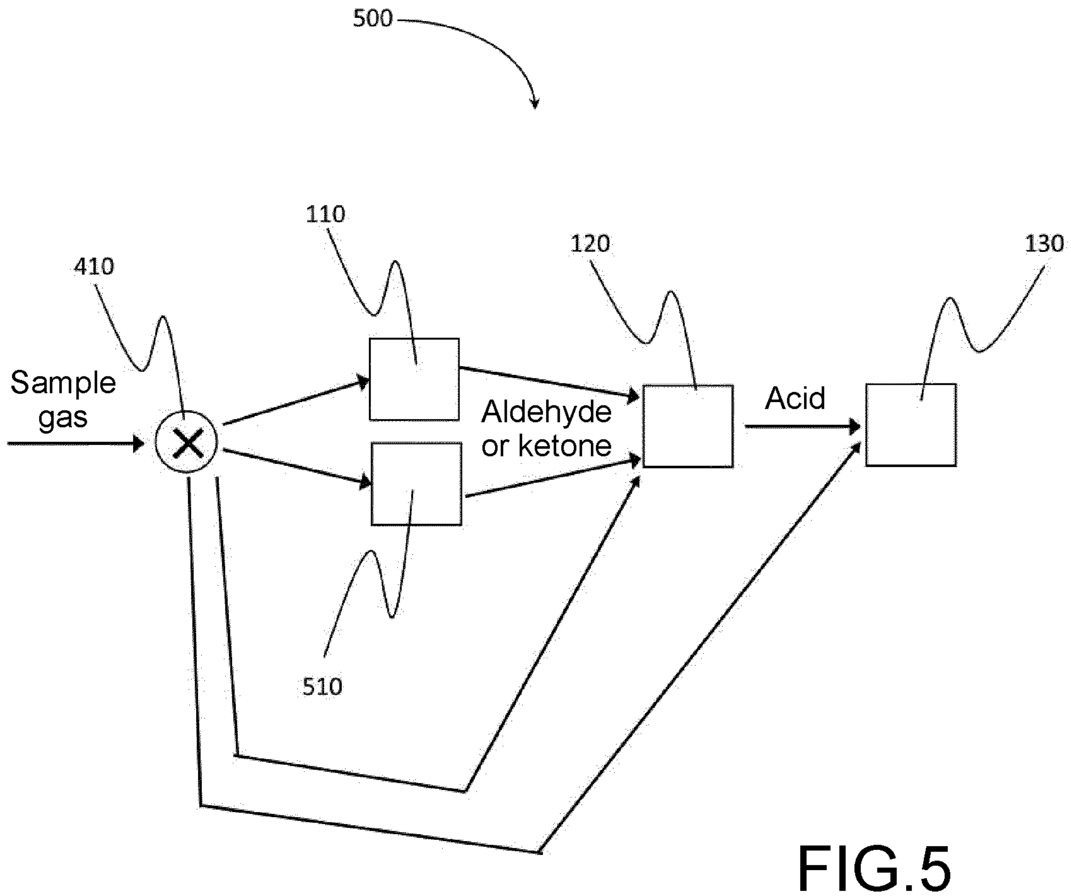
500
410
110
120
130
Sample gas
Aldehyde or ketone
Acid
510

FIG.5

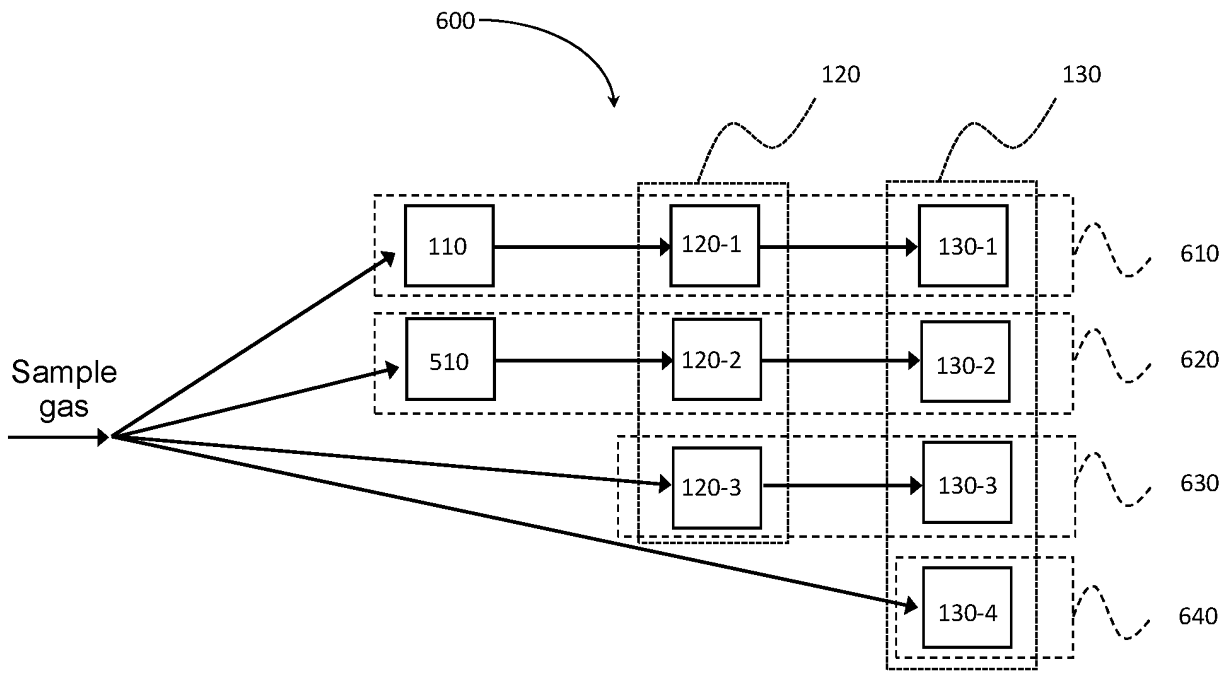
600
120
130
110
120-1
130-1
610
510
120-2
130-2
620
Sample gas
120-3
130-3
630
130-4
640

FIG.6

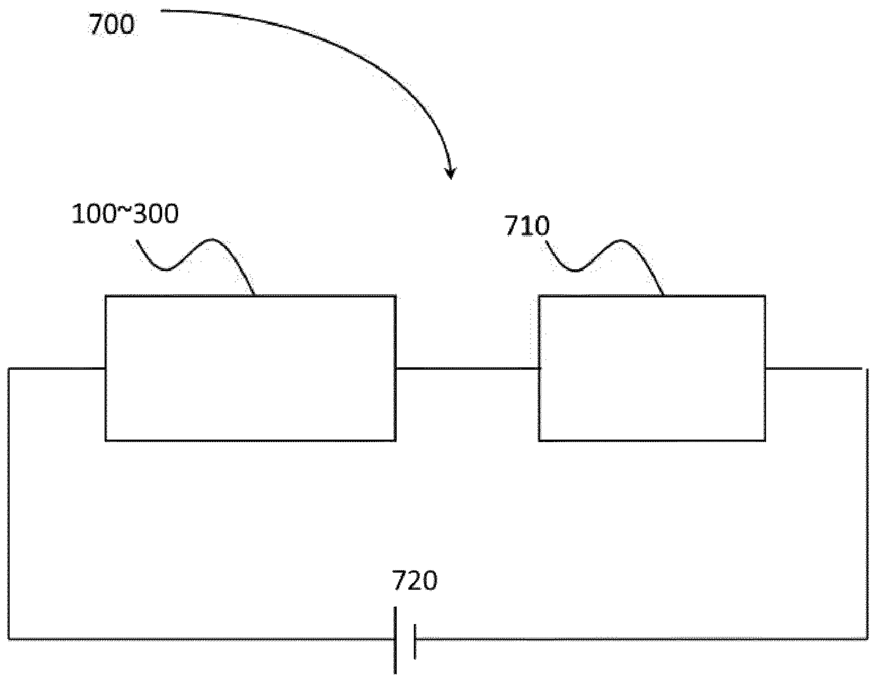


FIG.7

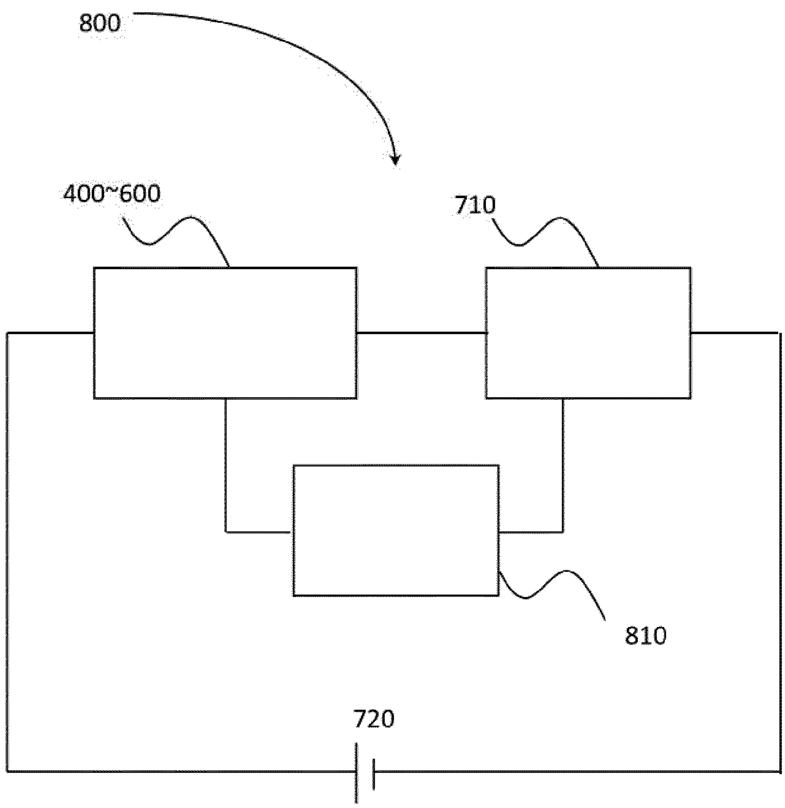


FIG.8

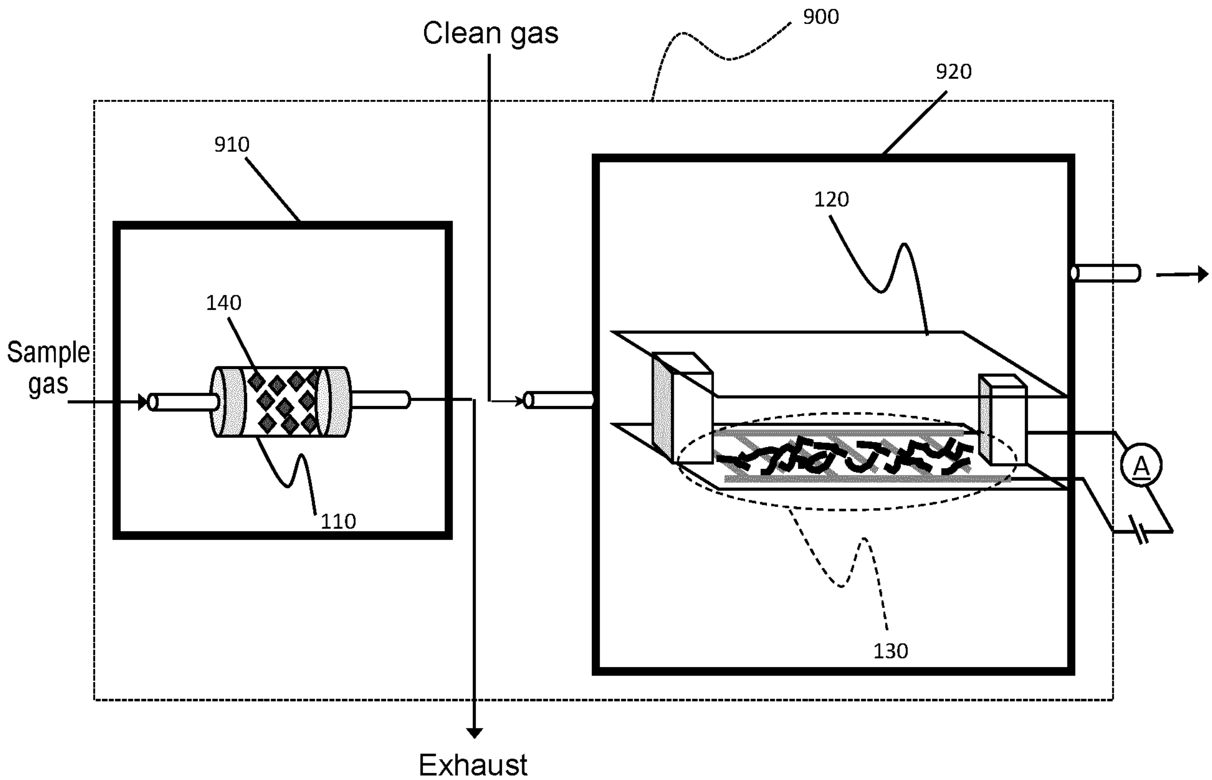
Clean gas
900
920
910
120
140
Sample gas
110
A
130
Exhaust

FIG.9A

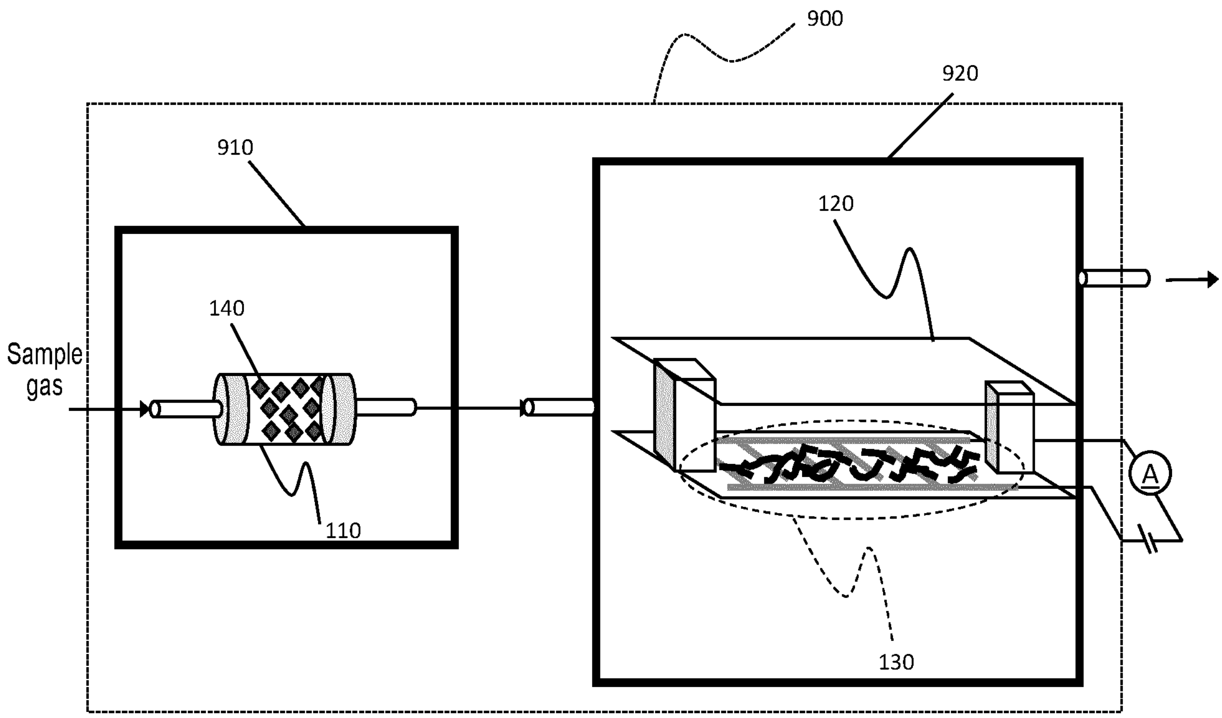
900
920
910
120
140
Sample gas
110
A
130

FIG.9B

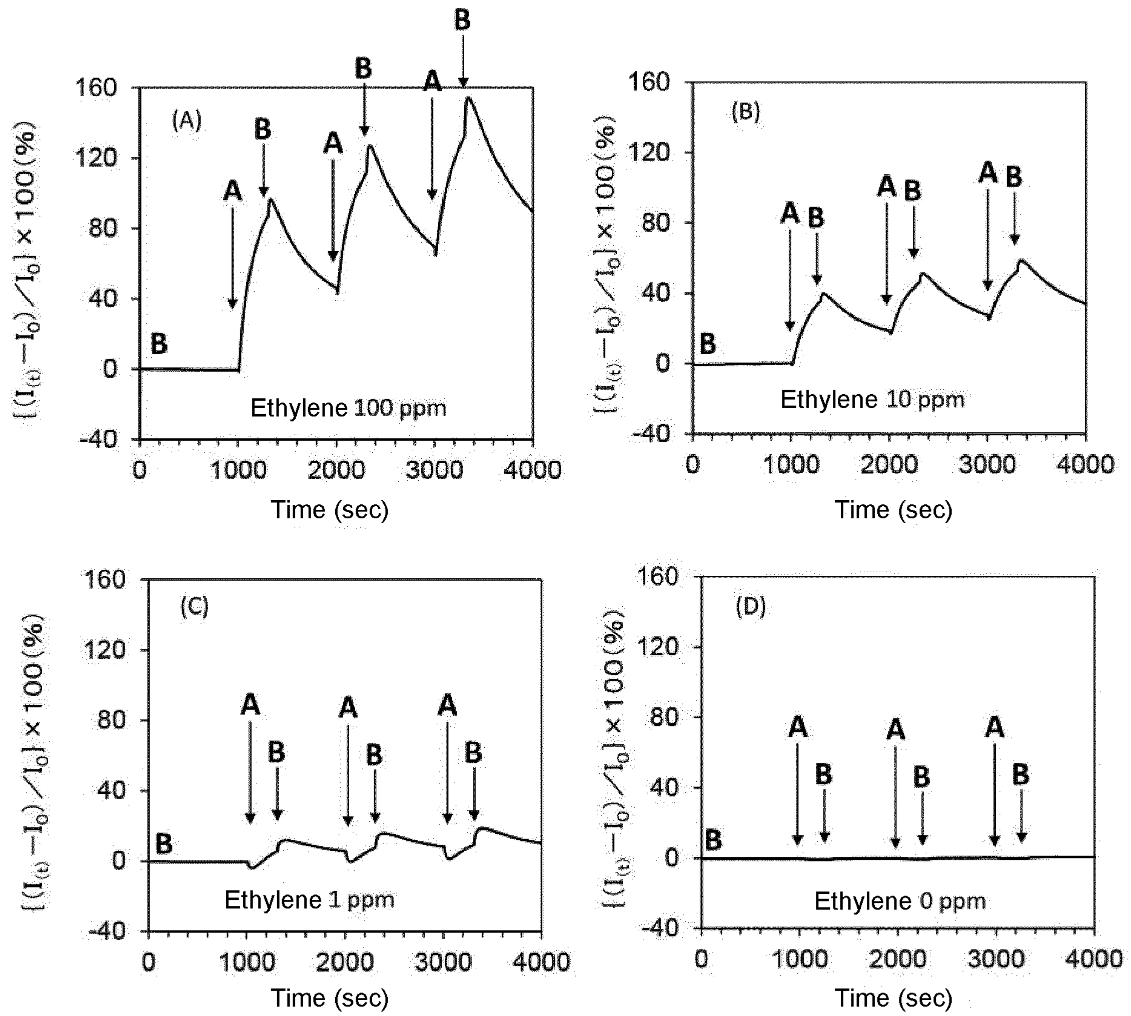
(A)
Ethylene 100 ppm
(B)
Ethylene 10 ppm
(C)
Ethylene 1 ppm
(D)
Ethylene 0 ppm
{(I(t) − I0)／I0} × 100 (%)
Time (sec)

FIG.10

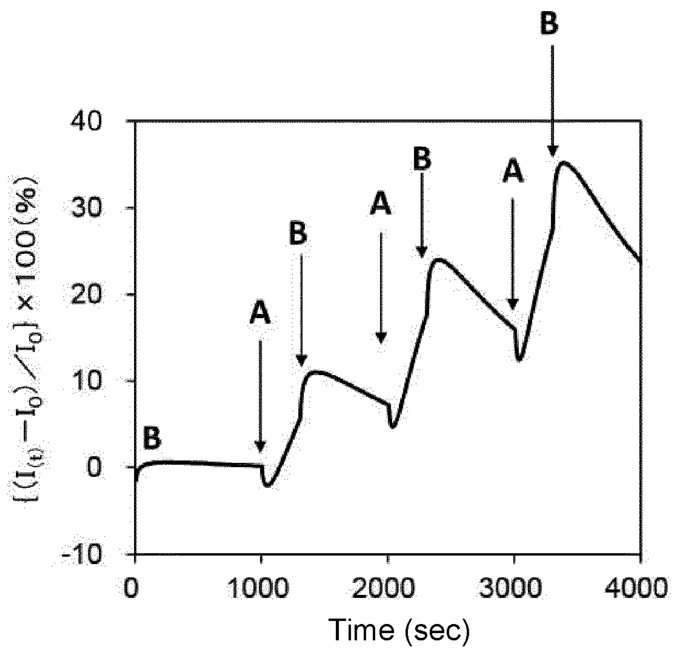
{(I(t) − I0)／I0} × 100 (%)
Time (sec)

FIG.11

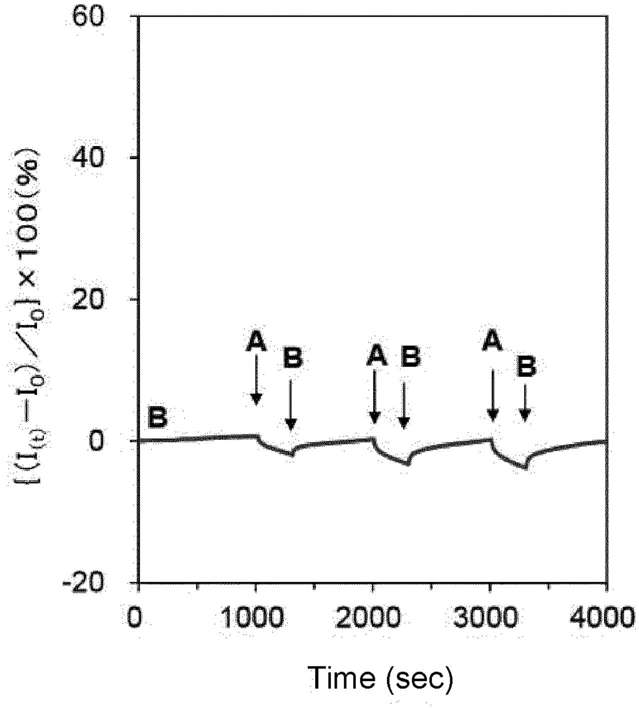
60
40
20
0
-20
0
1000
2000
3000
4000
Time (sec)
{(I(t)−I0)/I0}×100(%)
A
B
A
B
A
B
B

FIG.12

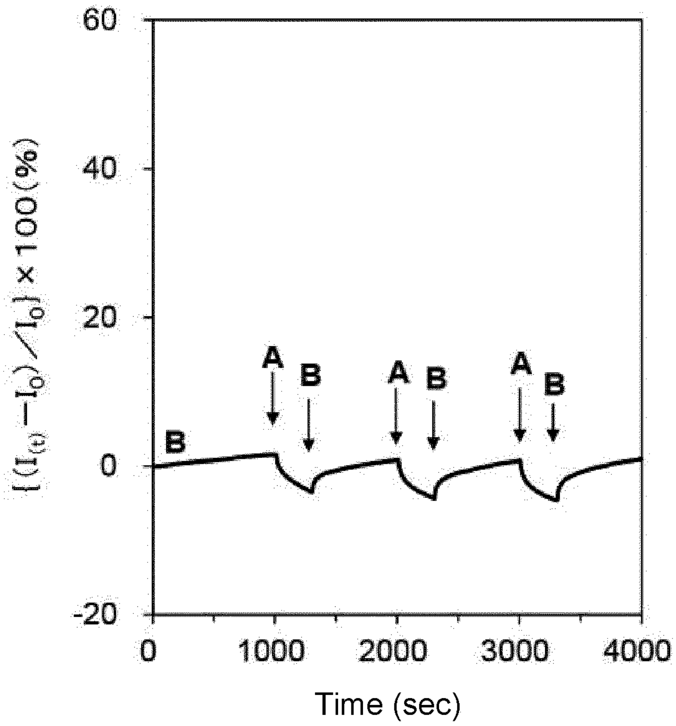
60
40
20
0
-20
0
1000
2000
3000
4000
Time (sec)
{(I(t)−I0)/I0}×100(%)
A
B
A
B
A
B
B

FIG.13

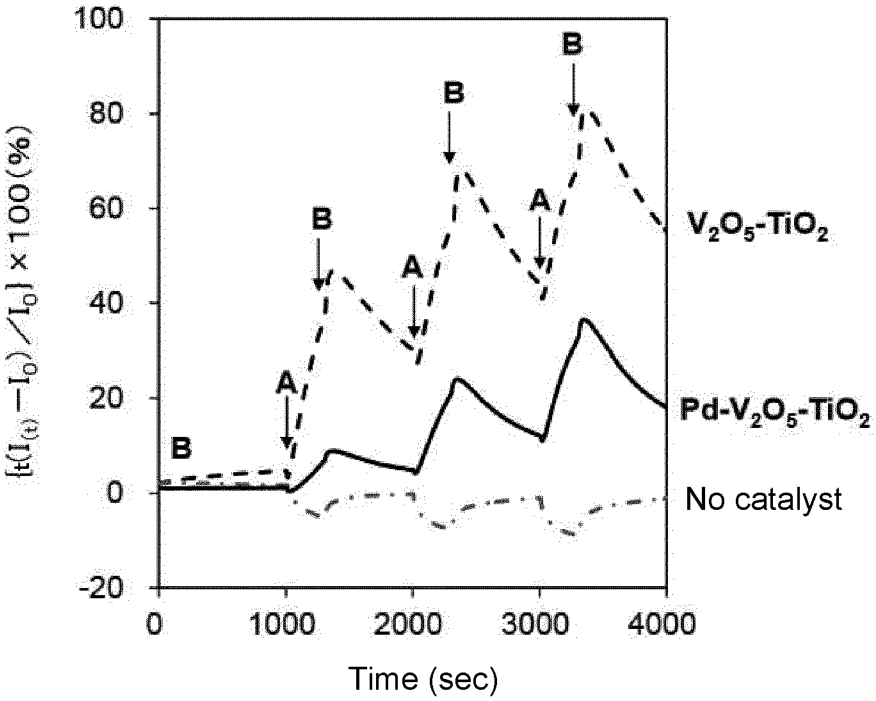
100
80
60
40
20
0
-20
0
1000
2000
3000
4000
Time (sec)
A
B
V2O5-TiO2
Pd-V2O5-TiO2
No catalyst

FIG.14

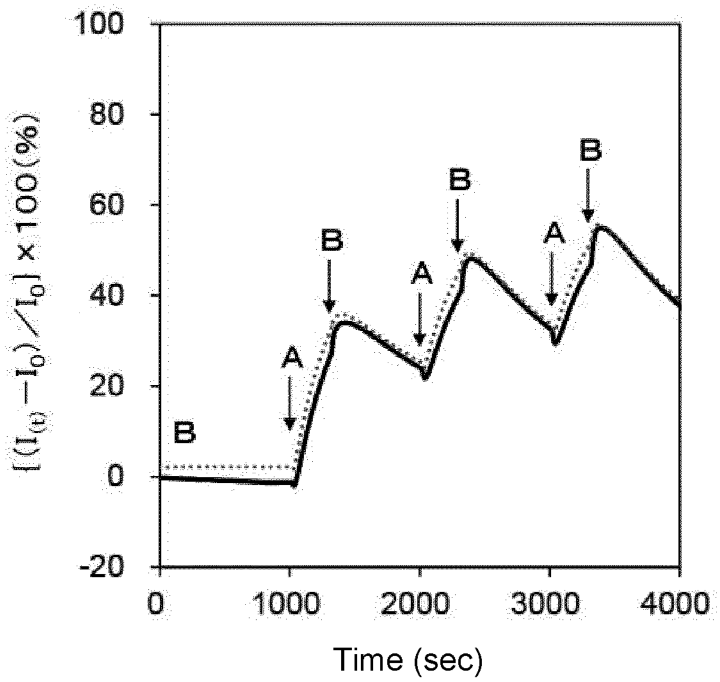
100
80
60
40
20
0
-20
0
1000
2000
3000
4000
Time (sec)
A
B

FIG.15

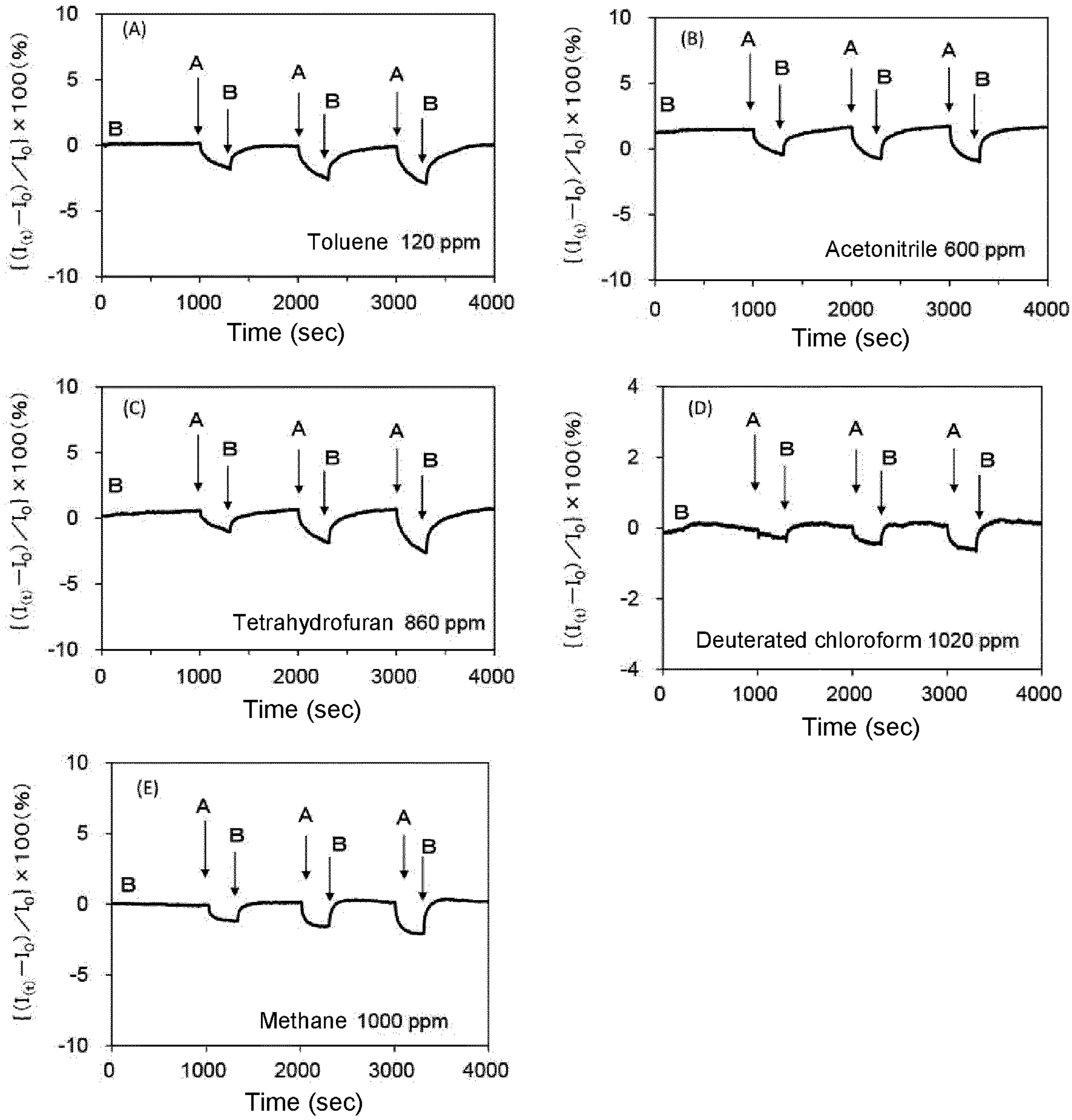
(A)
Toluene 120 ppm
(B)
Acetonitrile 600 ppm
(C)
Tetrahydrofuran 860 ppm
(D)
Deuterated chloroform 1020 ppm
(E)
Methane 1000 ppm
A
B
Time (sec)
{(I(t)−I0)/I0}×100(%)

FIG.16

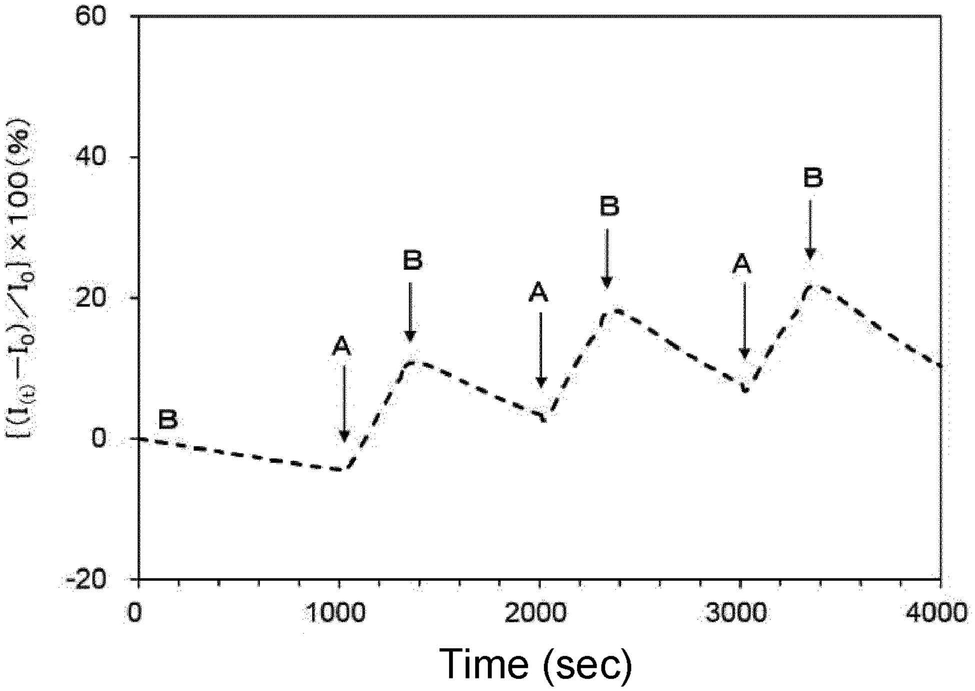
60
40
20
0
-20
{(I(t) − I0)／I0} × 100 (%)
B
A
B
A
B
A
B
0
1000
2000
3000
4000
Time (sec)

FIG.17

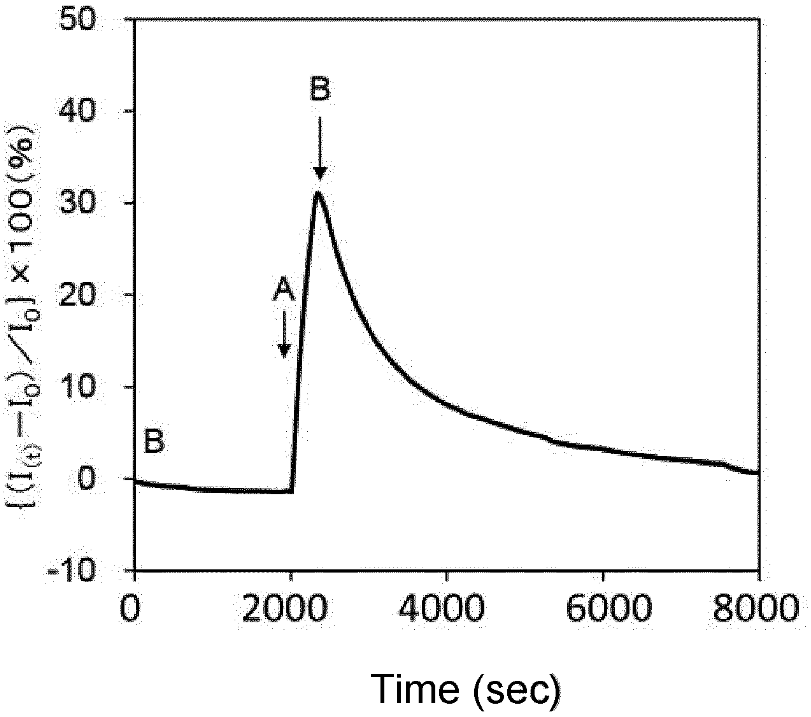
50
40
30
20
10
0
-10
{(I(t) − I0)／I0} × 100 (%)
B
A
B
0
2000
4000
6000
8000
Time (sec)

FIG.18

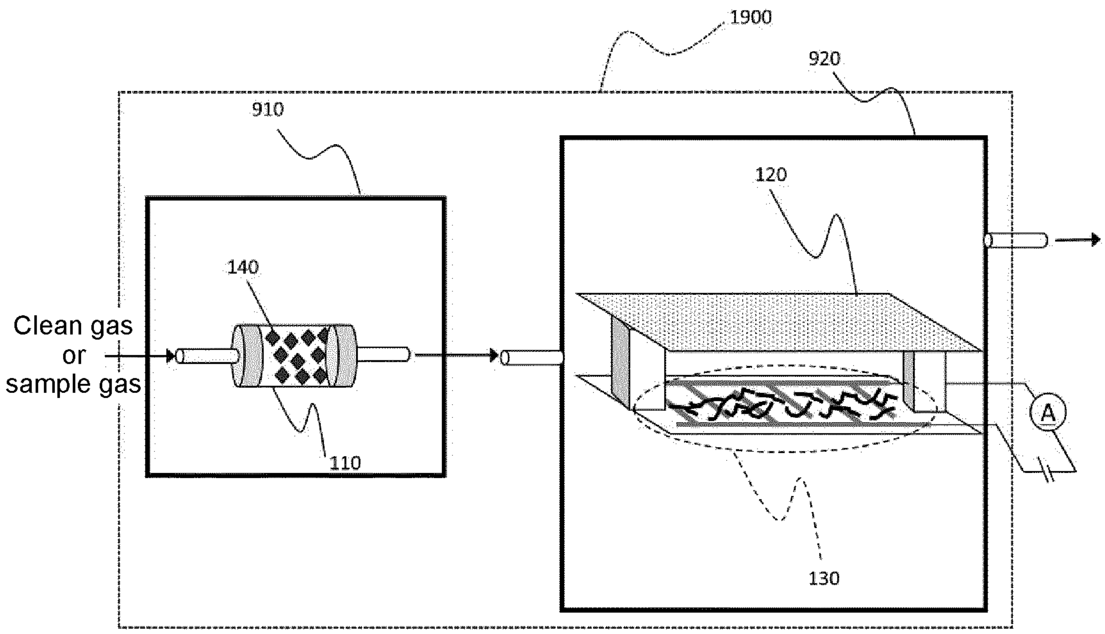
1900
920
910
120
140
Clean gas
or
sample gas
110
A
130

FIG.19

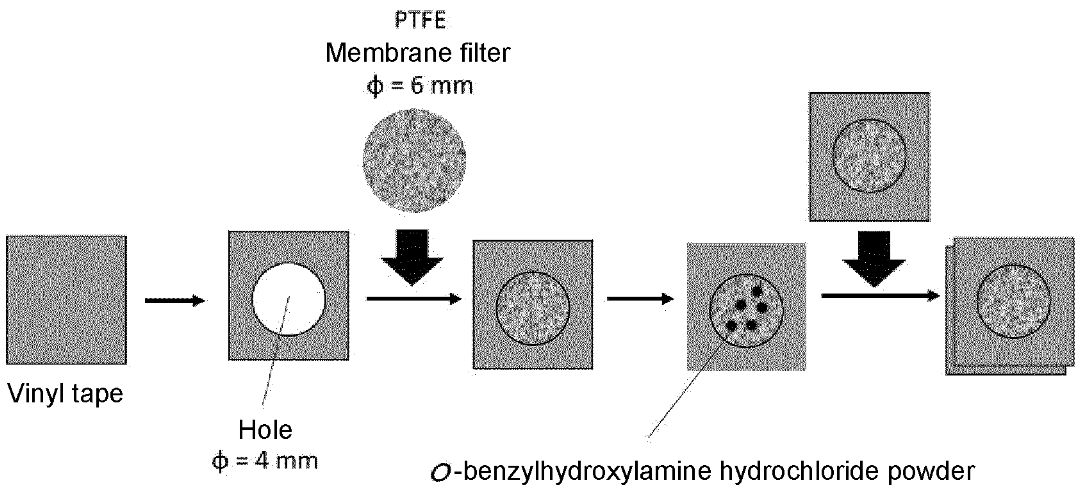
PTFE
Membrane filter
ϕ = 6 mm
Vinyl tape
Hole
ϕ = 4 mm
O-benzylhydroxylamine hydrochloride powder

FIG.20

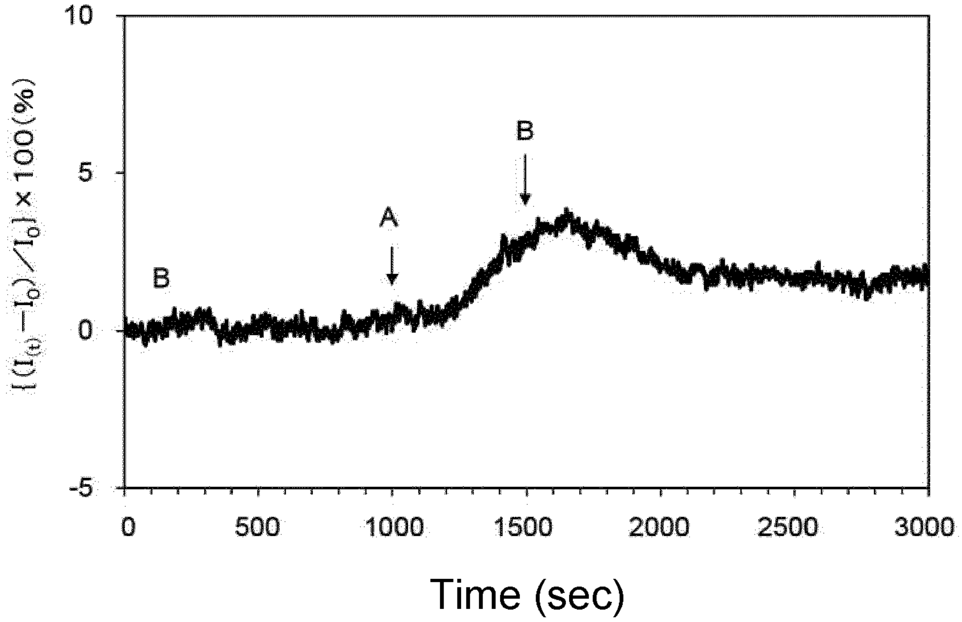
10
5
0
-5
{(I(t)−I0)／I0}×100(%)
B
A
B
0
500
1000
1500
2000
2500
3000
Time (sec)

FIG.21

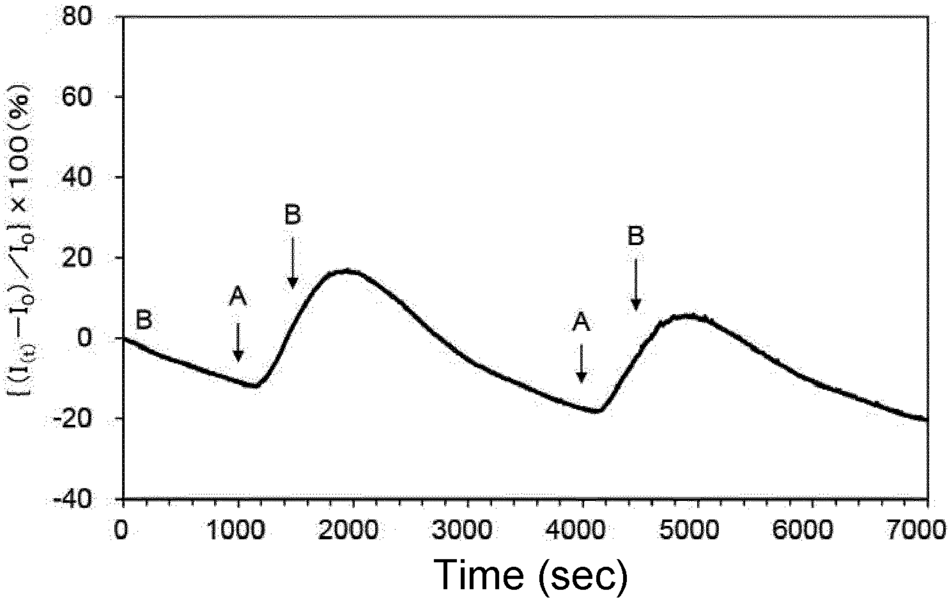
80
60
40
20
0
-20
-40
{(I(t)−I0)／I0}×100(%)
B
A
B
A
B
0
1000
2000
3000
4000
5000
6000
7000
Time (sec)

FIG.22

**INTERNATIONAL SEARCH REPORT**

International application No.
PCT/JP2020/039138

A. CLASSIFICATION OF SUBJECT MATTER
Int.Cl. B82Y30/00(2011.01)i, G01N27/12(2006.01)i, B01J23/648(2006.01)i
FI: G01N27/12B, G01N27/12C, G01N27/12M, B01J23/648Z, B82Y30/00

According to International Patent Classification (IPC) or to both national classification and IPC

B. FIELDS SEARCHED

Minimum documentation searched (classification system followed by classification symbols)
Int.Cl. G01N27/00-G01N27/24, G01N33/00, G01N33/497

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JSTChina/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | WO 2019/49693 A1 (NATIONAL INSTITUTE FOR MATERIALS SCIENCE) 14 March 2019 (2019-03-14), entire text, all drawings | 1-29 |
| A | JP 2017-521685 A (C2SENSE, INC.) 03 August 2017 (2017-08-03), paragraphs [0063]-[0069] | 1-29 |
| A | JP 2019-109087 A (CANON INC.) 04 July 2019 (2019-07-04), paragraphs [0091], [0121], [0122] | 1-29 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November 2020 | 17 November 2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/039138

| | | |
|---|---|---|
| WO 2019/49693 A1 | 14 March 2019 | (Family: none) |
| JP 2017-521685 A | 03 August 2017 | US 2017/0212104 A1<br>paragraphs [0061]-[0067]<br>WO 2016/010855 A1<br>EP 3170002 A1<br>CA 2955168 A1<br>CN 106687811 A |
| JP 2019-109087 A | 04 July 2019 | WO 2019/117039 A1 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013184222 A
- WO 2019049693 A


### Non-patent literature cited in the description


- **YOMOGIDA, Y. et al.** *Nat.Commun.,* 2016, vol. 7, 12056
- **ISHIHARA, S. et al.** *J.Am.Chem.Soc.,* 2016, vol. 138, 8221-8227
- **E.E.NESTEROV et al.** *Macromolecules,* 2014, vol. 47, 506-516
- **A.STAUBITZ et al.** *Organic Letter,* 2013, vol. 15, 4666-4669